# EUROPÄISCHE PATENTANMELDUNG

(11) **EP 0 648 761 A2**
(43) Veröffentlichungstag der Anmeldung: **19.04.1995**
(21) Anmeldenummer: 94112967.8
(22) Anmeldetag: 19.08.1994
(51) Int. Cl.: C07D 403/06, G03C 7/36, C07D 239/16

(54) **4-Pyrimidonverbindungen gelbkuppler für farbphotografisches Aufzeichnungsmaterial**

(30) Priorität: 01.09.1993 DE 4329418
(71) Anmelder: Agfa-Gevaert AG, D-51373 Leverkusen (DE)
(72) Erfinder: Bergthaller, Peter, Dr., D-51467 Bergisch Gladbach (DE)

(57) **Zusammenfassung**

Aus Quinazölan der Formel I werden durch chromogene Entwicklung gelbe Azomethinfarbstoffe besonders hoher Stabilität erhalten 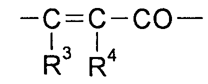
In Formel I bedeuten
- Q: einen Rest zur Vervollständigung eines 4-Pyrimidonringes;
- X: einen bei der chromogenen Entwicklung abspaltbaren Rest;
- R¹: Alkyl mit 1 bis 6 C-Atomen;
- R²: Halogen, -CF₃, Alkyl, Aryl, Alkoxy, Aryloxy, Alkylthio, Arylthio, Acylamino, Sulfonamido, Sulfuramido, Carbamoyl, Alkoxycarbonyl, Sulfonyl, Sulfamoyl oder einen heterocyclischen Rest, wobei zwei benachbarte Reste R² einen Ring vervollständigen können;
- n: eine ganze Zahl von 1 bis 4.

## Beschreibung

Gegenstand der Erfindung sind neue Gelbkuppler für farbfotografische Aufzeichnungsmaterialien und farbfotografische Aufzeichnungsmaterialien, die die neuen Gelbkuppler enthalten. Gegenstand der Erfindung sind auch Bilder, die gelbe Azomethinfarbstoffe besonders hoher Stabilität aus den neuen Gelbkupplern enthalten.

Die neuen Gelbkuppler entsprechen der allgemeinen Formel I 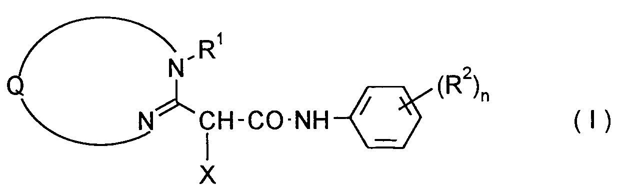
worin bedeuten
- Q: einen Rest zur Vervollständigung eines 4-Pyrimidonringes;
- X: einen bei der chromogenen Entwicklung abspaltbaren Rest;
- R¹: Alkyl mit 1 bis 6 C-Atomen;
- R²: Halogen, -CF₃, Alkyl, Aryl, Alkoxy, Aryloxy, Alkylthio, Arylthio, Acylamino, Sulfonamido, Sulfuramido, Carbamoyl, Alkoxycarbonyl, Sulfonyl, Sulfamoyl oder einen heterocyclischen Rest, wobei zwei benachbarte Reste R² einen Ring vervollständigen können;
- n: eine ganze Zahl von 1 bis 4.

Der durch Q dargestellte Rest zur Vervollständigung eines 4-Pyrimidonringes ist insbesondere eine Gruppe der Formel 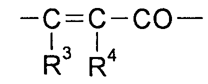
worin R³ und R⁴ einen Rest zur Vervollständigung eines 5- bis 7-gliedrigen Ringes bedeuten, z.B. eines Benzol-, Pyrazol- oder Cyclohexenringes, oder worin einer der Reste R³ und R⁴ für Wasserstoff, Alkyl, Aryl oder Alkoxy, der andere für -CN, Acylamino, Carbamoyl, Alkoxycarbonyl, Sulfonyl oder eine Phosphorestergruppe steht.

Der durch X dargestellte bei der chromogenen Entwicklung abspaltbare Rest ist beispielsweise ein Halogenatom, insbesondere -Cl, oder eine über ein Sauerstoffatom oder ein Ringstickstoffatom angeknüpfte cyclische Gruppe. In besonders bevorzugten Ausführungsformen der Erfindung bedeutet X einen 1,2,3- oder 1,2,4-Triazolrest.

Ein durch R¹ oder R² dargestellter oder in einem der durch R² dargestellten Substitenten enthaltener Alkylrest kann geradkettig oder verzweigt, unsubstituiert oder substituiert sein. Mögliche Substituenten sind beispielsweise -OH, Alkoxy oder Aryloxy.

Es ist bevorzugt, wenn mindestens einer der durch R², R³ und R⁴ dargestellten Substituenten oder ein Substituent an einem durch R³ und R⁴ vervollständigten Ring einen längerkettigen Rest mit diffüsionsverhindernder Wirkung enthält oder mit einem Polymergerüst verknüpft ist.

Beispiele für erfindungsgemäße Kuppler sind im folgenden angegeben. 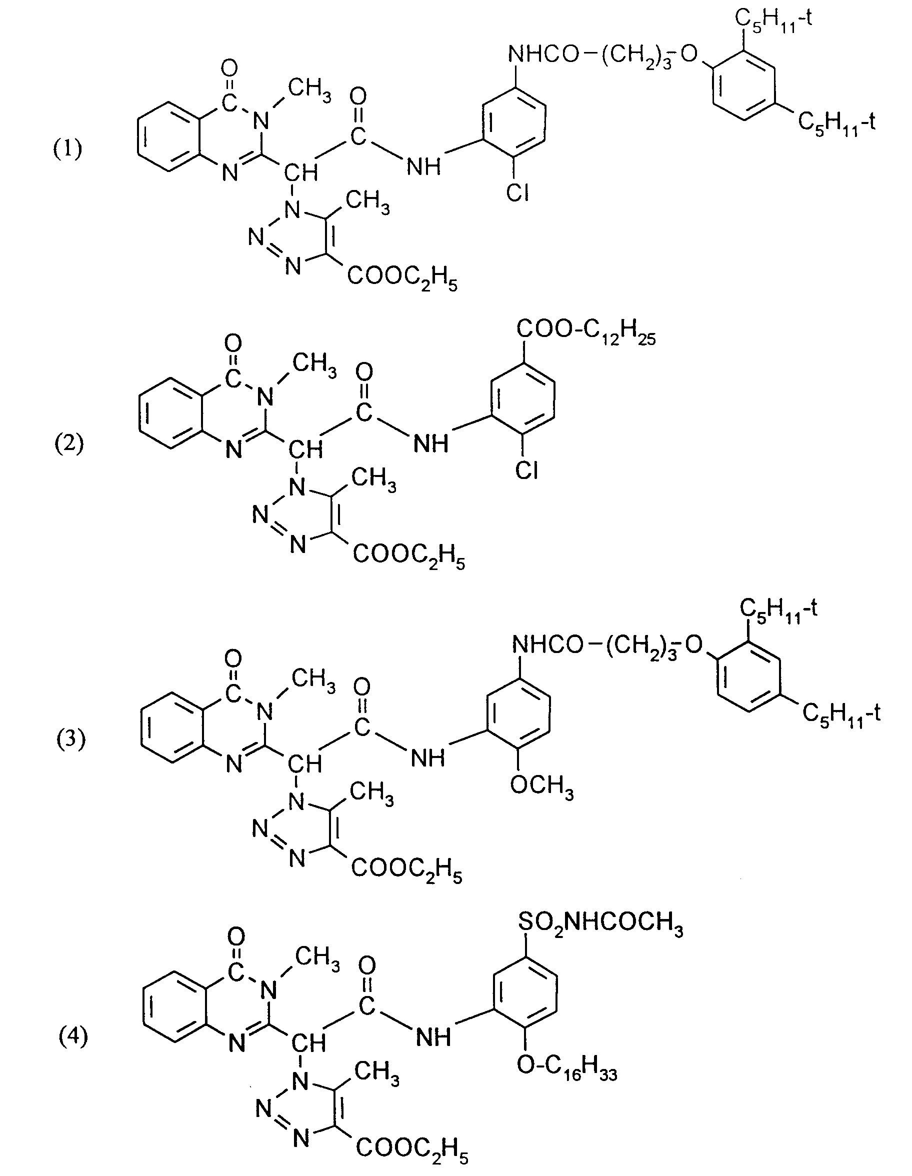 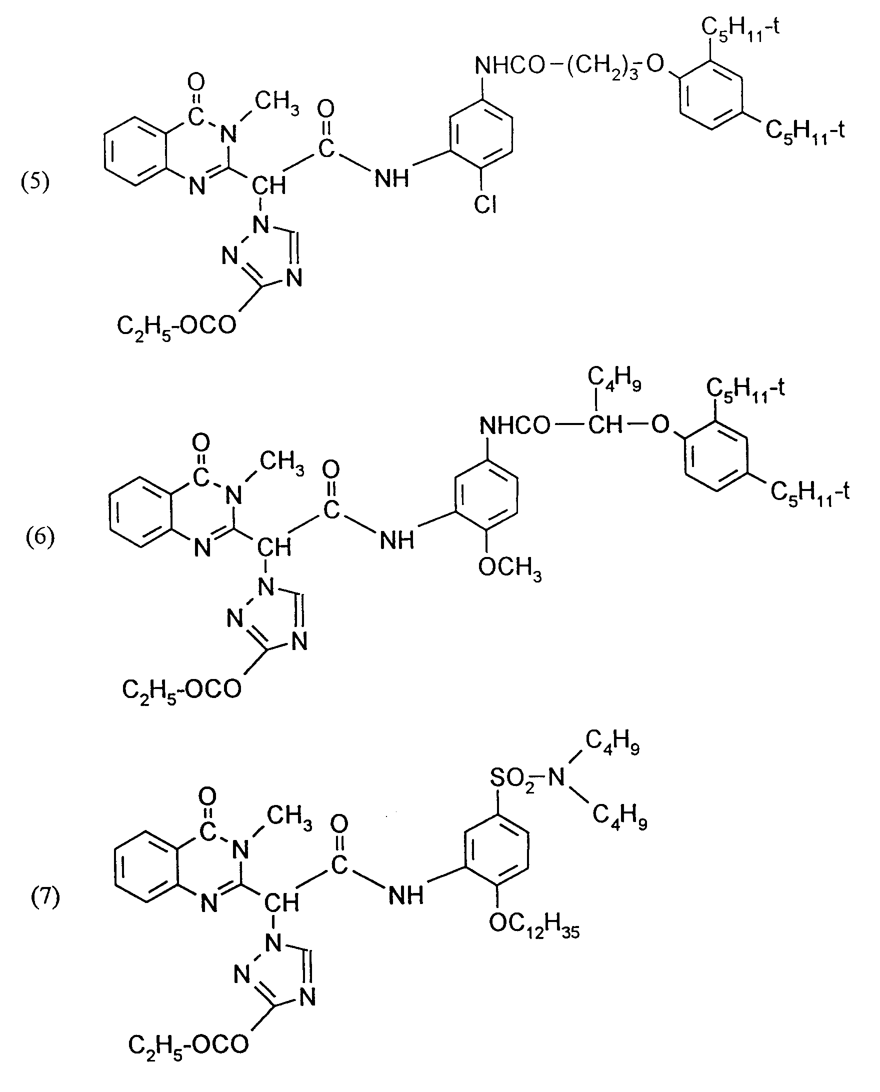 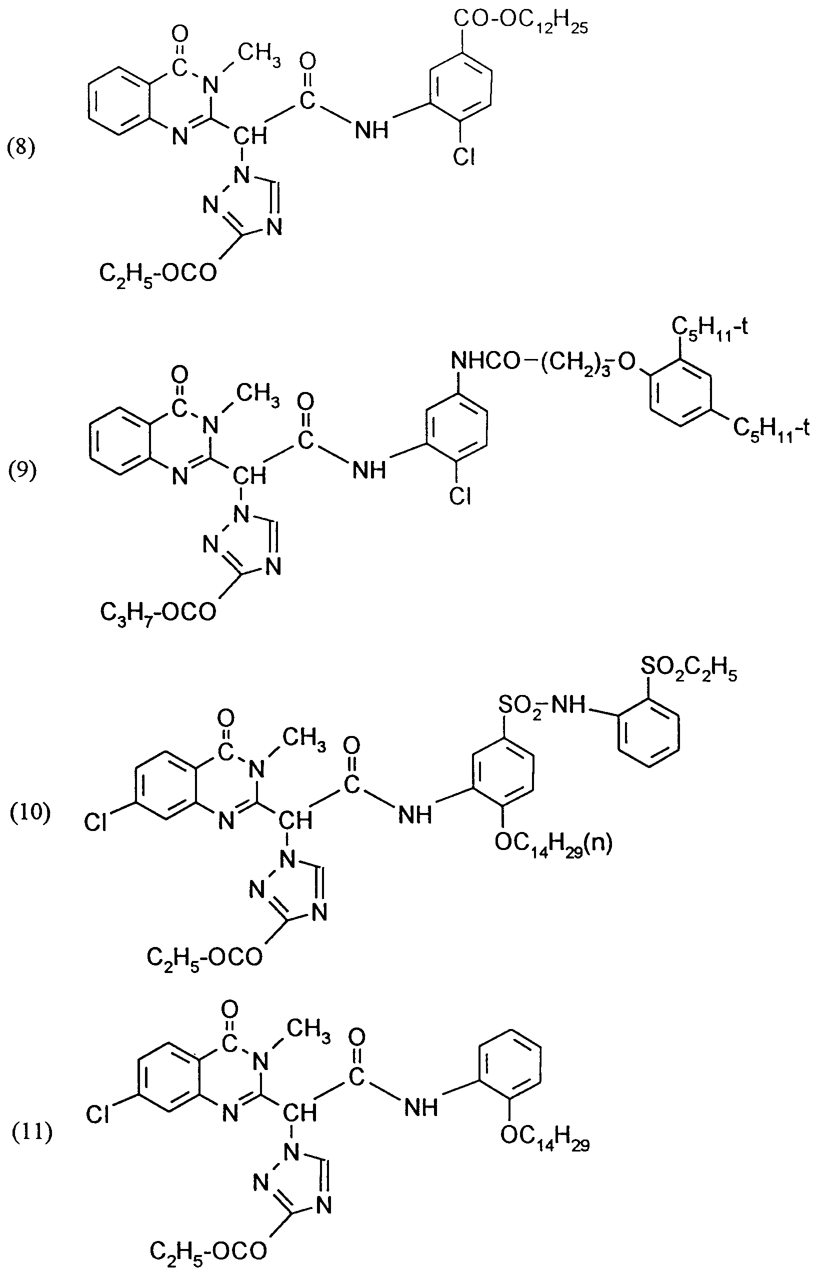 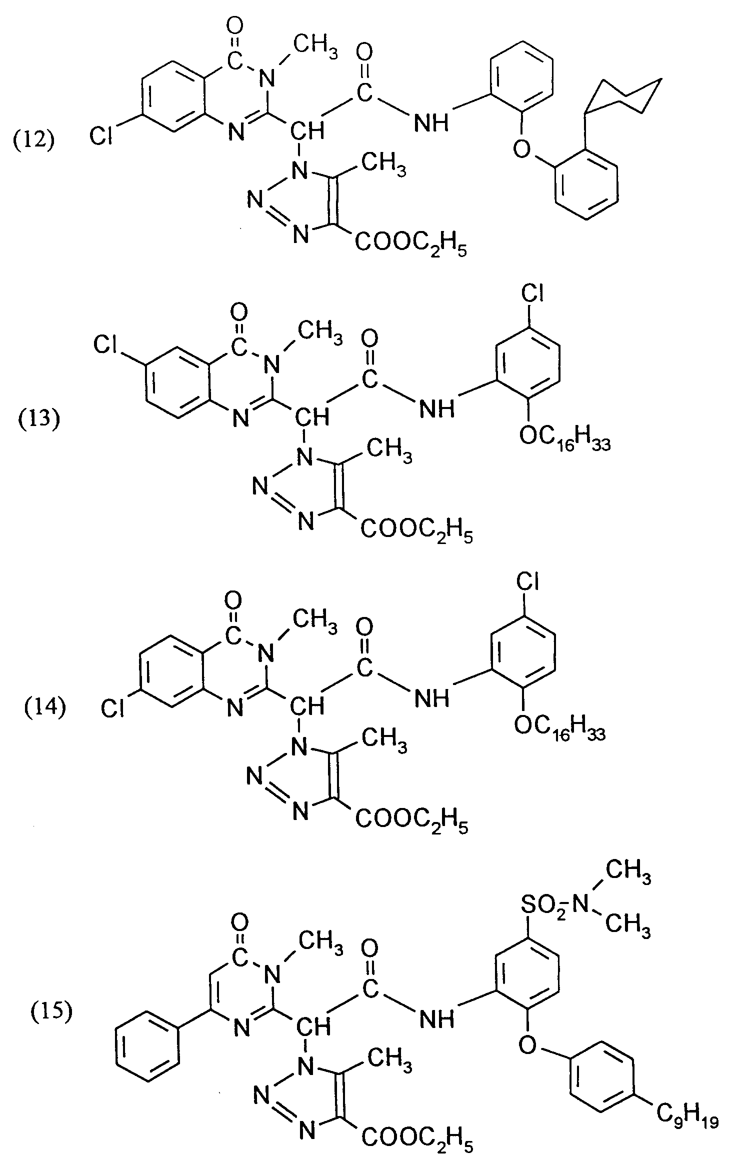 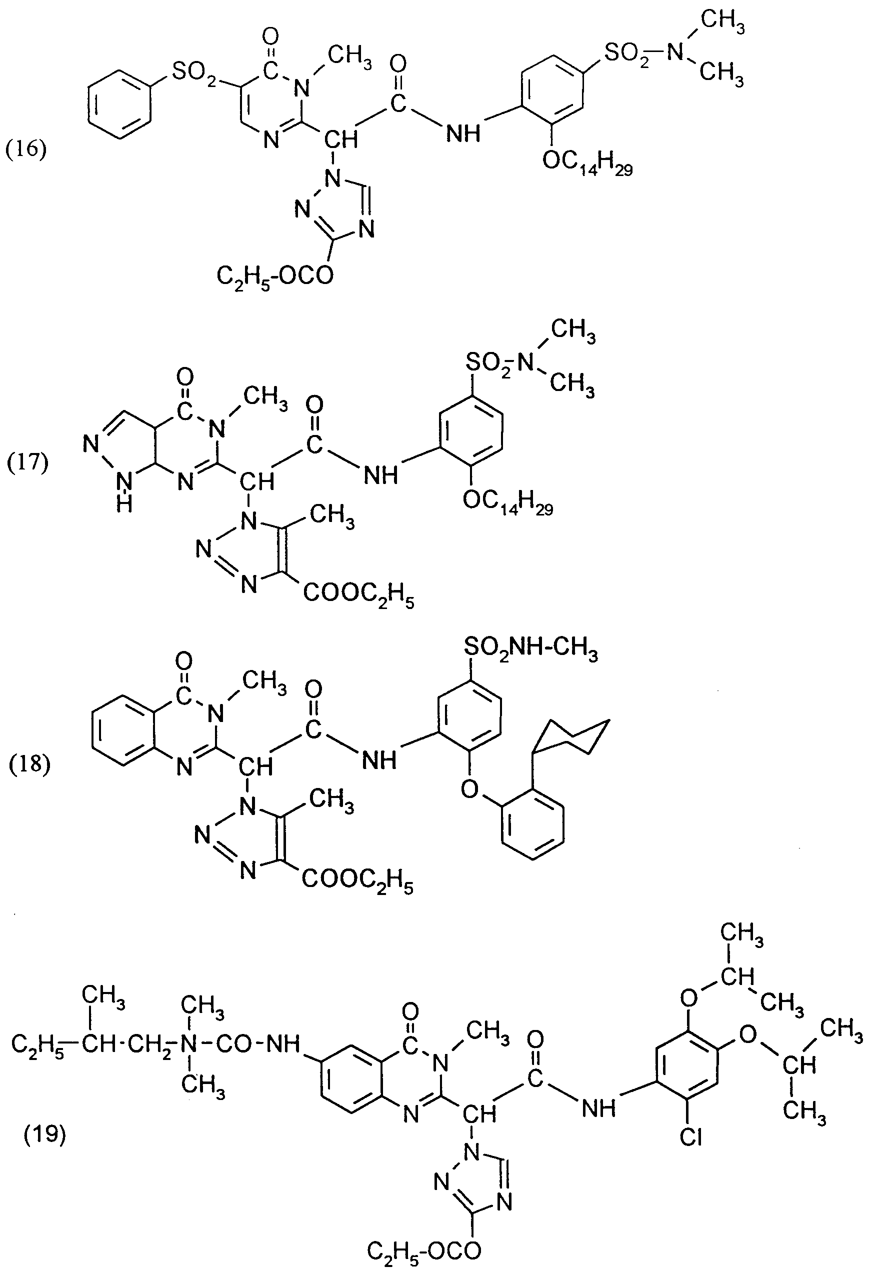 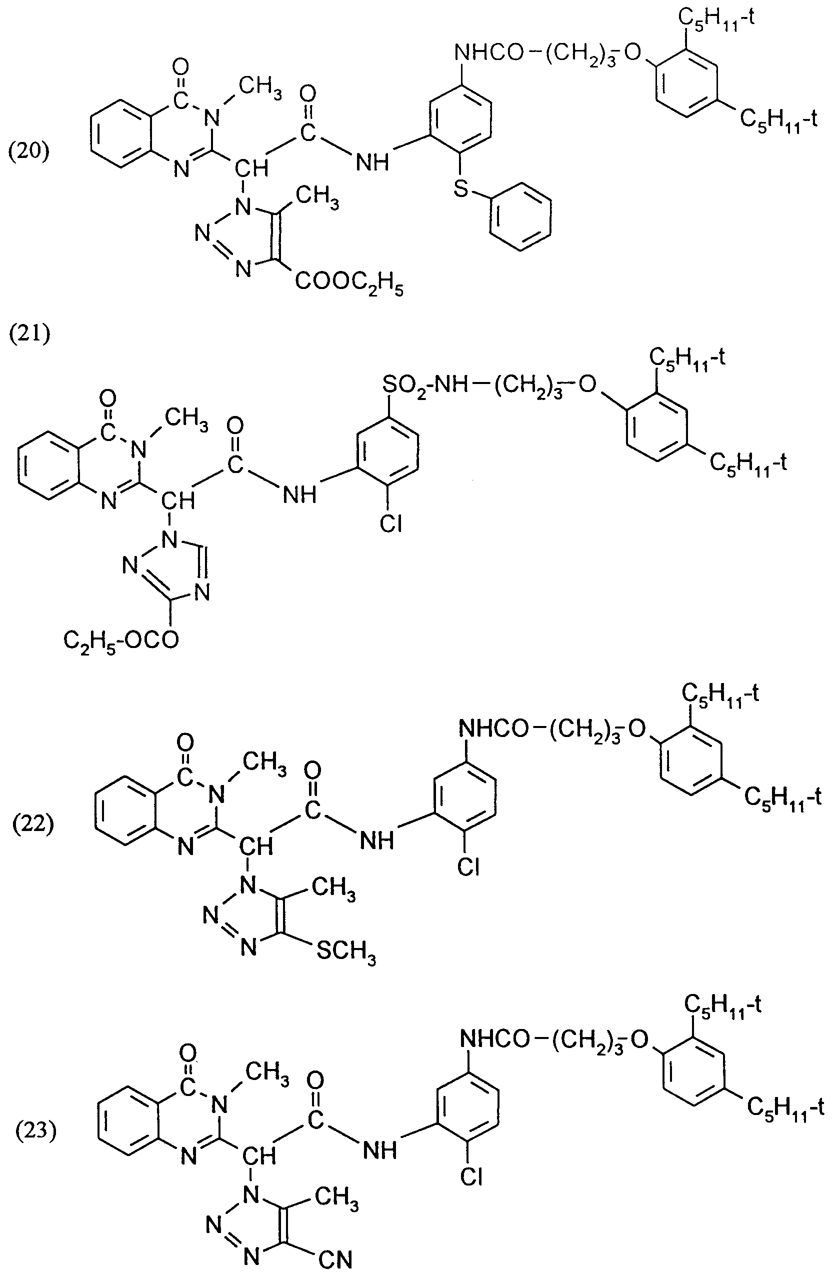 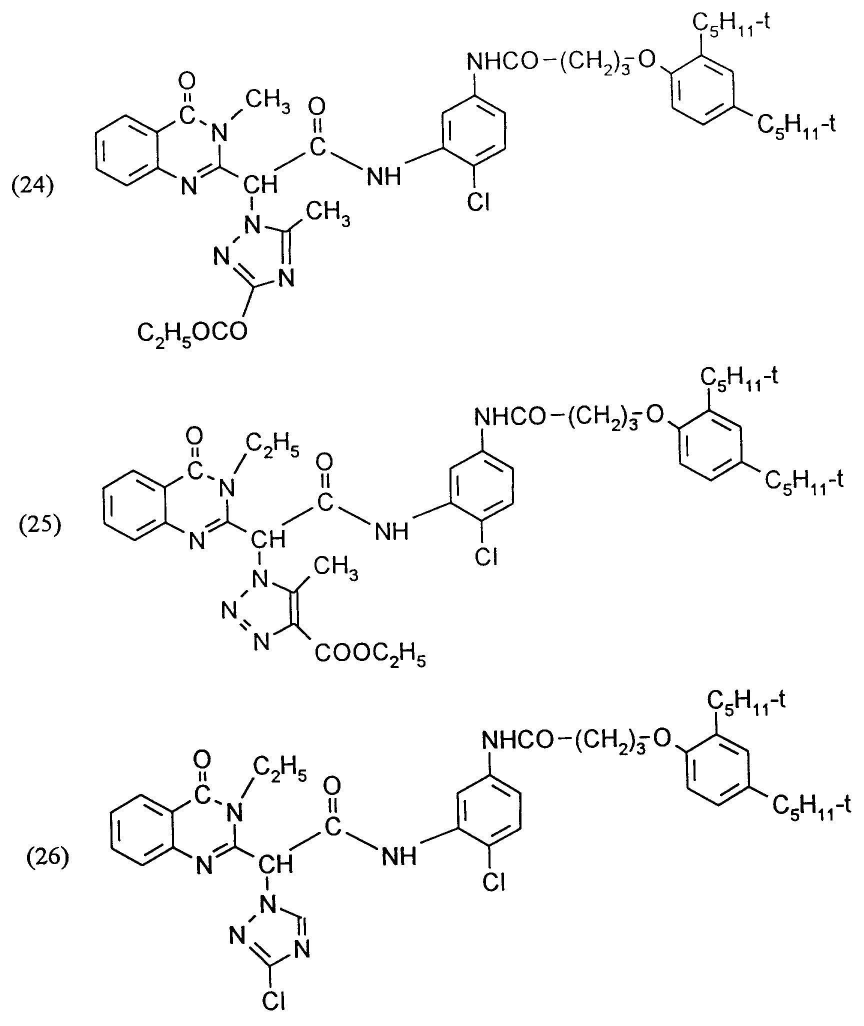 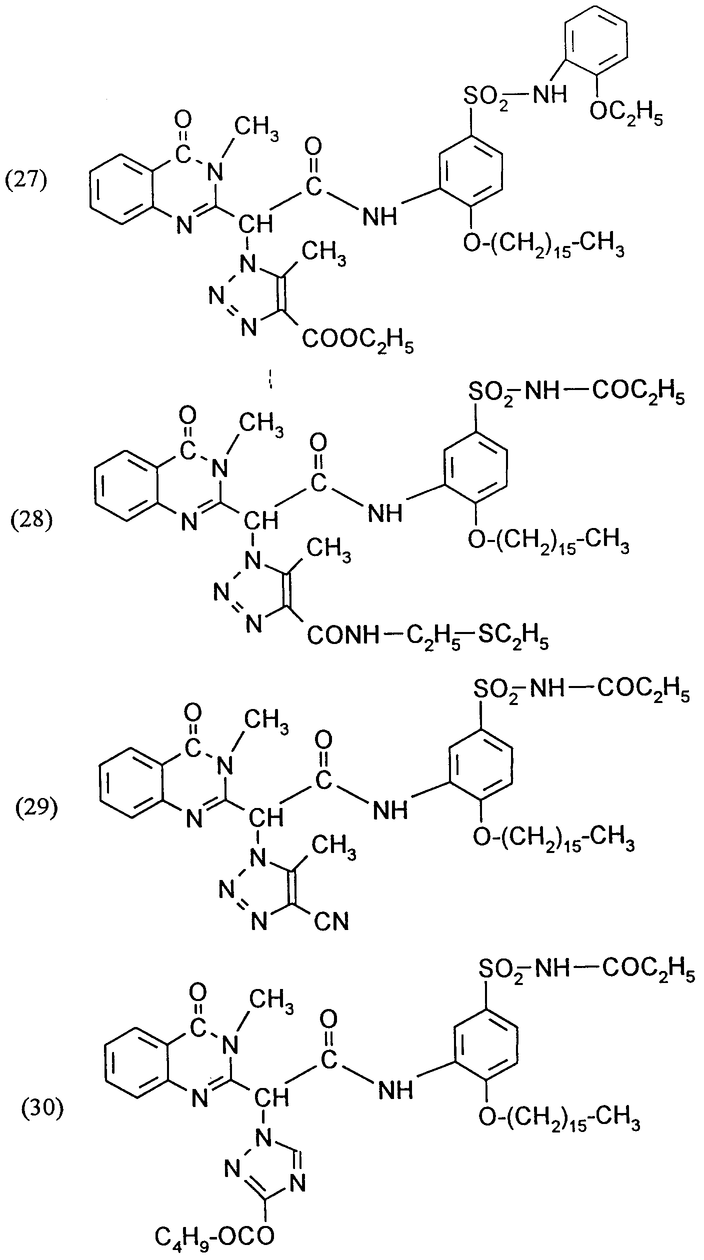 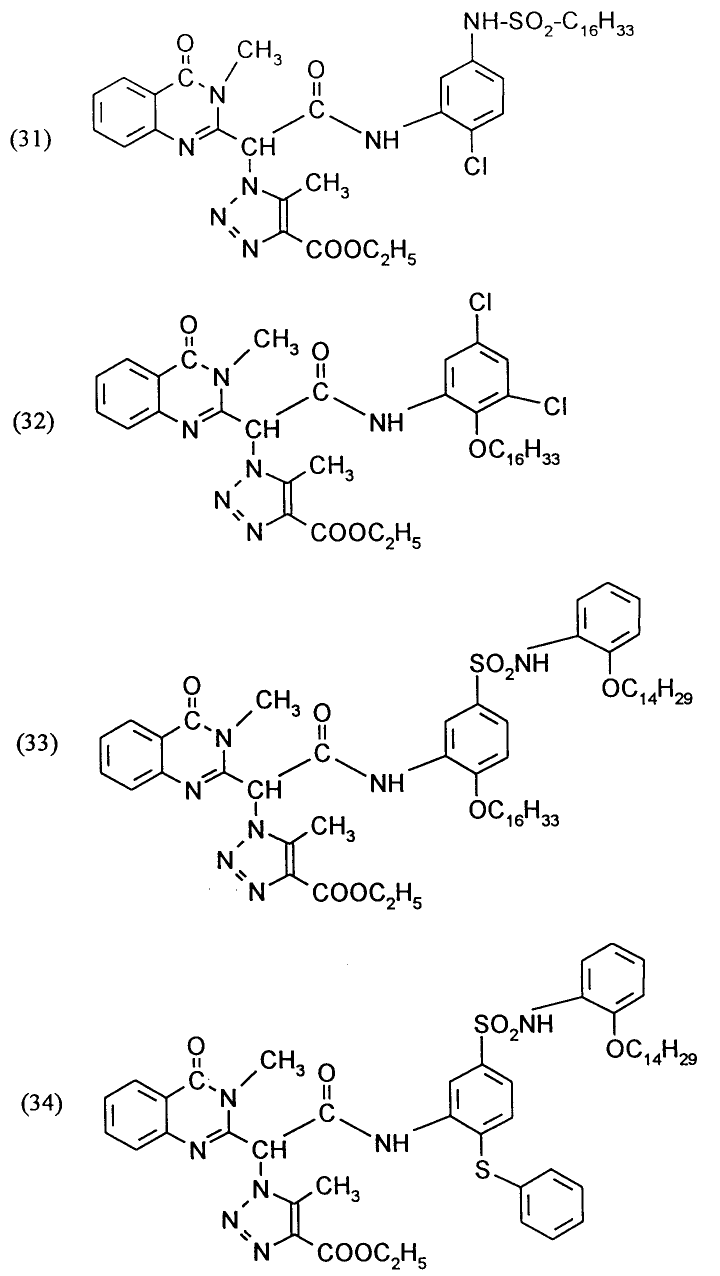 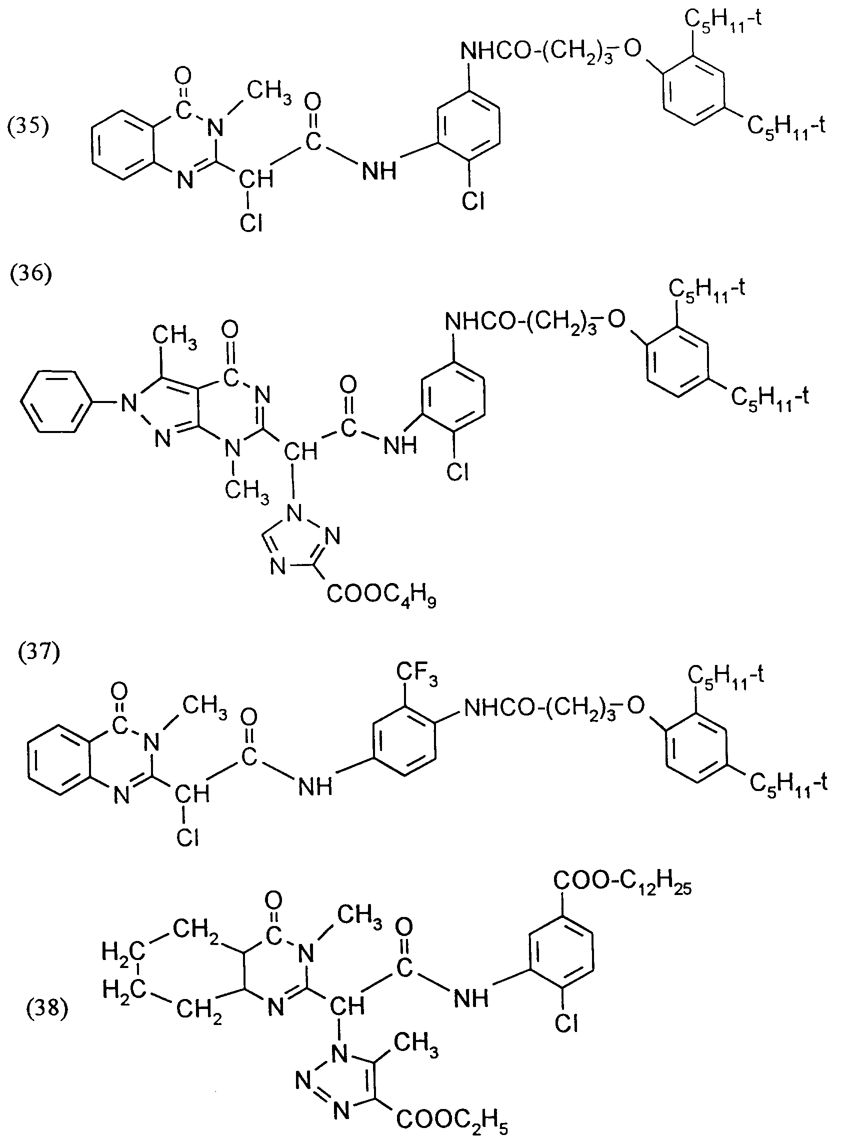
Die Herstellung der neuen Gelbkuppler läßt sich besonders einfach am Beispiel der 3-Alkylchinazolon-2-essigsäureanilide darstellen:
2-Aminobenzoesäure-N-methylamid (Anthranilsäuremethylamid) wird durch Umsetzung von Isatosäureanhydrid (1-H-Benzoxazin-2,4-dion) mit wäßriger Methylaminlösung hergestellt.

Aus substituierten 2-Aminobenzamiden wird z.B. durch Umsetzen mit einem Maloniminoetherhydrochlorid, das aus dem entsprechenden Cyanessigsäureester oder Cyanessigsäureanilid mit HCl und Ethanol bei 0°C erhalten wird, oder mit einem Malonanilid-iminothioether das Chinazolon-2-essigsäure-derivat hergestellt. Im Fall der N-alkylierten Chinazolonessigester kann die Einführung der Anilidgruppe auf besonders einfache Weise durch Verschmelzen bei Temperaturen zwischen 145 und 170°C erfolgen. Das Verfahren ist in der Gelbkupplerchemie allgemein üblich und in DE-A-37 11 418 genau beschrieben. Ein bevorzugtes Lösungsmittel dafür ist Chlorbenzol.

Die Einführung von nukleophilen Fluchtgruppen erfolgt im allgemeinen durch Austausch einer guten Abgangsgruppe, z.B. eines Chlor- oder Bromatoms oder einer Selenoxidgruppe, gegen das entsprechende Neukleophil, bevorzugt in Gegenwart einer Hilfsbase.

Folgende Beispiele dienen zur Erläutetung der allgemeinen Hinweise zur Herstellung der neuen Gelbkuppler.

### 3-Methylchinazolin-4-on-2-essigsäure-ethylester:

Zu einer auf 80°C erhitzten Lösung von 30 g (0,2 mol) Anthranilsäuremethylamid in 150 ml Isopropanol trägt man in drei Portionen unter Rühren 42 g (0,215 mol) Malonsäureethylester-iminoethylether-hydrochlorid und hält noch 40 min unter Rückfluß. Anschließend gießt man auf 300 g Eis, läßt 1 Stunde stehen und saugt ab. Ausbeute 50 g weiße Nadeln mit dem Schmelzpunkt: 105-107°C.

### 3-Methylchinazolin-4-on-2-essigsäure-2-chlor-5-[4(2,4-di-t-pentylphenoxy)-butyryl]amino-anilid (Vieräquivalentkuppler)

Man erhitzt 24 g 3-Methylchinazolin-4-on-2-essigsäure-ethylester und 45 g 2-Chlor-5-[4(2,4-di-t-pentylphenoxy)-butyryl]aminoanilid in 50 ml 1,2-Dichlorbenzol 90 min auf 155°C und treibt das freigesetzte Ethanol mit einem schwachen Stickstoffstrom ab. Anschließend gießt man auf 250 ml Acetonitril und laßt 5 Tage zur Kristallisation stehen. Man saugt ab, wäscht mit Acetonitril nach und trocknet an der Luft. Ausbeute 36 g Schmelzpunkt: 173-177°C.

### 3-Methylchinazolin-4-on-2-bromessigsäure-2-chlor-5-[4(2,4-di-t-pentylphenoxy)butyryl]amino-anilid (bromierter Kuppler)

Man erhitzt 30 g Vieräquivalentkuppler in 200 ml Essigsäure auf 95°C, kühlt rasch auf 35°C und tropft unter intensivem Rühren 8 g Brom in 30 ml Essigsäure zu. Man rührt 15 min weiter und fällt den bromierten Kuppler mit 45 g Eis aus. Man dekantiert, wäscht mit viel Wasser und digeriert mit 100 ml Acetonitril. Ausbeute: 29 g, Schmelzpunkt: 200-204°C.

### Erfindungsgemäßer Kuppler 1:

Man löst 7 g des bromierten Kupplers in 100 ml Ethylacetat, gibt 3 g 4-Methyl-1,2,3-triazol-5-carbonsäure-ethylester zu und tropft bei 25°C 3 ml Tetramethylguanidin zu. Man rührt 1 Stunde weiter, gibt 40 ml 10 %ige Salzsäure zu, trennt die wäßrige Phase ab, wäscht die organische Phase mit Wasser, trocknet mit Magnesiumsulfat, dampft im Wasserstrahlvakuum ein, digeriert mit Hexan und kristallisiert aus Acetonitril um. Man erhält Kuppler 1 der Erfindung als Gemisch zweier Isomeren mit dem Schmelzbereich: 145-155°C. Ausbeute: 5 g.

Die neuen Kuppler sind ausgezeichnet stabil, zeigen keinerlei Neigung zum Zerfall im Sinn der Säurespaltung, die insbesondere bei Benzoylacetaniliden oft zum Wirkungsverlust führt.

Sie ergeben Gelbfarbstoffe mit ausgezeichneter Stabilität insbesondere gegen hydrolytischen Abbau im Sinn des "dark fading". Sie sind daher sowohl für die Herstellung fotografischer Durchsichtsbilder oder Negative als auch für Aufsichtsbilder hervorragend geeignet.

Mit den neuen Kupplern können Materialien zur Herstellung fotografischer Aufsichtsbilder hergestellt werden, bei denen die blauempfindliche Schicht abweichend vom Stand der Technik als oberste der lichtempfindlichen Schichten angordnet ist. Die oft beträchtliche UV-Absorption des Restkupplers der blauempfindlichen Schicht kann daher mit zum Schutz der darunterliegenden Magenta- und Cyanteilbilder gegen UV-Strahlung beitragen.

Die aus den neuen Kupplern gebildeten Gelbfarbstoffe zeigen im allgemeinen Absorptionsmaxima bei etwa 450 nm, die langwelliger liegen als die der aus vergleichbarer Pivaloylacetaniliden gebildeten Gelbfarbstoffe. Die Gelbfarbstoffe wirken aber visuell trotzdem nicht röter, weil ihre langwellige Bandenflanke außerordentlich steil abfällt. Daher zeigen sie auch in hohen Dichten keine Eintrübung zu lehmigen oder nach orange verschobenen Nuancen.

Die neuen Gelbkuppler sind in den üblichen Kupplerlösungen, z.B. hochsiedenden Estern oder Carbonamiden oder Phosphorsäureestern ausgezeichnet löslich und ergeben auch bei hohem Kuppler-Ölbildnerverhältnis stabile und kristallisationsbeständige Dispersionen bzw. Emulgate. Die neuen Gelbkuppler können auch in Form von beladenen Latices oder ölbildnerfreien Dispersionen eingesetzt werden.

Die neuen Gelbkuppler können zur Einstellung einer definierten Kupplungskinetik gemeinsam mit bekannten Gelbkupplern in Abmischungen ohne Nachteile verwendet werden.

Einer im Rahmen der Erfindung bevorzugten Verbindungsklasse, den Chinazolon-2-essigsäureaniliden liegt eine Struktur zugrunde, die allerdings in - im Chinazolonring - nicht alkylierter Form bereits aus DE-A 37 11 418 bekannt ist. Die dort genannten Verbindungen geben bei der chromogenen Kupplung aber im allgemeinen keine ansprechenden Gelbfarbstoffe, dafür zeigen sie hervorragende Eignung als zur Abspaltung von fotografisch nützlichen Gruppen geeignete funktionelle Kuppler, insbesondere als DIR-Kuppler.

Es ist überraschend, daß die Alkylierung eines N-Atomes, ausreicht, eine Kurzverschiebung der Absorption des Azomethinfarbstoffes zu bewirken, die zu Klaren Gelbnuancen führt, die weder grünbetont noch verrötet wirken. Der Austausch des ankondensierten Benzolringes gegen eine andere der genannten Gruppierungen, z.B. einen Pyrazol- oder Imidazolring, kann allerdings eine Erhöhung oder Verminderung des Extinktionskoeffizienten oder eine unbeträchtliche Verschiebung der Nuance hervorrufen.

Beispiele für farbfotografische Materialien sind insbesondere Farbnegativfilme, Farbumkehrfilme und farbfotografische Papiere.

Geeignete Träger zur Herstellung farbfotografischer Materialien sind z.B. Filme und Folien von halbsynthetischen und synthetischen Polymeren, wie Cellulosenitrat, Celluloseacetat, Cellulosebutyrat, Polystyrol, Polyvinylchlorid, Polyethylenterephthalat und Polycarbonat und mit einer Barytschicht oder α-Olefinpolymerschicht (z.B. Polyethylen) lamimiertes Papier. Diese Träger können mit Farbstoffen und Pigmenten, beispielsweise Titandioxid, gefärbt sein. Die Oberfläche des Trägers wird im allgemeinen einer Behandlung unterzogen, um die Adhäsion der fotografischen Emulsionsschicht zu verbessern, beispielsweise einer Corona-Entladung mit nachfolgendem Antrag einer Substratschicht. Erfindungsgemäß bevorzugt wird ein lichtreflektierender Träger.

Wesentliche Bestandteile der fotografischen Emulsionsschichten sind Bindemittel, Silberhalogenidkörnchen und Farbkuppler.

Als Bindemittel wird vorzugsweise Gelatine verwendet. Diese kann jedoch ganz oder teilweise durch andere synthetische, halbsynthetische oder auch natürlich vorkommende Polymere ersetzt werden.

Die Bindemittel verfügen in der Regel über eine ausreichende Menge an funktionellen Gruppen, so daß durch Umsetzung mit geeigneten Härtungsmitteln genügend widerstandsfähige Schichten erzeugt werden können. Solche funktionellen Gruppen sind insbesondere Aminogruppen, aber auch Carboxylgruppen, Hydroxylgruppen und aktive Methylengruppen.

Das als lichtempfindlicher Bestandteil in dem fotografischen Material befindliche Silberhalogenid kann als Halogenid Chlorid, Bromid oder Iodid bzw. Mischungen davon enthalten. Beispielsweise kann der Halogenidanteil wenigstens einer Schicht zu 0 bis 15 mol-% aus Iodid, zu 0 bis 100 mol-% aus Chlorid und zu 0 bis 100 mol-% aus Bromid bestehen. Im Falle von Farbnegativ- und Farbumkehrfilmen werden üblicherweise Silberbromidiodidemulsionen, im Falle von Farbnegativ- und Farbumkehrpapier üblicherweise Silberchloridbromidemulsionen mit hohem Chloridanteil bis zu reinen Silberchloridemulsionen verwendet. Erfindungsgemäß bevorzugt werden Silberhalogenidemulsionen, deren Halogenidanteil zu 95 mol-% oder mehr, vorzugsweise zu 99 mol-% oder mehr aus Chlorid bestehen. Es kann sich um überwiegend kompakte Kristalle handeln, die z.B. regulär kubisch oder oktaedrisch sind oder Übergangsformen aufweisen können. Vorzugsweise können aber auch plättchenförmige Kristalle vorliegen, deren durchschnittliches Verhältnis von Durchmesser zu Dicke bevorzugt wenigstens 5:1 ist, wobei der Durchmesser eines Kornes definiert ist als der Durchmesser eines Kreises mit einem Kreisinhalt entsprechend der projizierten Fläche des Kornes. Die Schichten können aber auch tafelförmige Silberhalogenidkristalle aufweisen, bei denen das Verhältnis von Durchmesser zu Dicke wesentlich größer als 5:1 ist, z.B. 12:1 bis 30:1.

Die Silberhalogenidkörner können auch einen mehrfach geschichteten Kornaufbau aufweisen, im einfachsten Fall mit einem inneren und einem äußeren Kornbereich (core/ shell), wobei die Halogenidzusammensetzung und/oder sonstige Modifizierungen, wie z.B. Dotierungen der einzelnen Kornbereiche unterschiedlich sind. Die mittlere Korngröße der Emulsionen liegt vorzugsweise zwischen 0,2 m und 2,0 m, die Korngrößenverteilung kann sowohl homo- als auch heterodispers sein. Homodisperse Korngrößenverteilung bedeutet, daß 95 % der Körner nicht mehr als ± 30% von der mittleren Korngröße abweichen.

Es können zwei oder mehrere Arten von Silberhalogenidemulsionen, die getrennt hergestellt werden, als Mischung verwendet werden.

Die Emulsionen können in der üblichen Weise chemisch oder spektral sensibilisiert sein und die Emulsionsschichten wie auch andere nicht-lichtempfindliche Schichten können in der üblichen Weise mit bekannten Härtungsmitteln, insbesondere mit carboxylgruppenaktivierenden Härtungsmitteln wie Carbamoylpyridiniumsalzen (z.B. gemäß DE-A-22 25 230, DE-A-23 17 677, DE-A-24 39 551), gehärtet sein.

Üblicherweise enthalten farbfotografische Silberhalogenidmaterialien mindestens je eine Silberhalogenidemulsionsschicht für die Aufzeichnung von Licht jedes der drei Spektralbereiche Rot, Grün und Blau. Zu diesem Zweck können die fotografischen Emulsionen unter Verwendung von Methinfarbstoffen oder anderen Farbstoffen spektral sensibilisiert werden. Besonders geeignete Farbstoffe sind Cyaninfarbstoffe, Merocyaninfarbstoffe und komplexe Merocyaninfarbstoffe.

Eine Übersicht über die als Spektralsensibilisatoren geeigneten Polymethinfarbstoffe, deren geeignete Kombinationen und supersensibilisierend wirkenden Kombinationen enthält Research Disclosure 17643 (Dez. 1978), Kapitel IV.

Insbesondere sind die folgenden Farbstoffe - geordnet nach Spektralgebieten - geeignet:
1. als Rotsensibilisatoren
   9-Ethylcarbocyanine mit Benzthiazol, Benzselenazol oder Naphthothiazol als basische Endgruppen, die in 5- und/oder 6-Stellung durch Halogen, Methyl, Methoxy, Carbalkoxy, Aryl substituiert sein können sowie 9-Ethyl-naphthoxathia- bzw. -selenacarbocyanine und 9-Ethyl-naphthothiaoxa- bzw. -benzimidazocarbocyanine, vorausgesetzt, daß die Farbstoffe mindestens eine Sulfoalkylgruppe am heterocyclischen Stickstoff tragen.
2. als Grünsensibilisatoren
   9-Ethylcarbocyanine mit Benzoxazol, Naphthoxazol oder einem Benzoxazol und einem Benzthiazol als basische Endgruppen sowie Benzimidazocarbocyanine, die ebenfalls weiter substituiert sein können und ebenfalls mindestens eine Sulfoalkylgruppe am heterocyclischen Stickstoff enthalten müssen.
3. als Blausensibilisatoren
   symmetrische oder asymmetrische Benzimidazo-, Oxa-, Thia- oder Selenacyanine mit mindestens einer Sulfoalkylgruppe am heterocyclischen Stickstoff und gegebenenfalls weiteren Substituenten am aromatischen Kern, sowie Apomerocyanine mit einer Rhodaningruppe.

Auf Sensibilisatoren kann verzichtet werden, wenn für einen bestimmten Spektralbereich die Eigenempfindlichkeit des Silberhalogenids ausreichend ist, beispielsweise die Blauempfindlichkeit von Silberbromiden.

Den unterschiedlich sensibilisierten Emulsionsschichten werden nicht diffundierende niedermolekulare oder polymere Farbkuppler zugeordnet, die sich in der gleichen Schicht oder in einer dazu benachbarten Schicht befinden können. Gewöhnlich werden den rotempfindlichen Schichten Cyankuppler, den grünempfindlichen Schichten Magentakuppler und den blauempfindlichen Schichten Gelbkuppler zugeordnet. Im vorliegenden Fall ist der oder den blauempfindlichen Schichten ein Gelbkuppler der Formel I zugeordnet.

Farbkuppler zur Erzeugung des blaugrünen Teilfarbenbildes sind in der Regel Kuppler vom Phenol- oder α-Naphtholtyp; geeignete Beispiele hierfür sind (Formeln II, III, IV, V, VI) 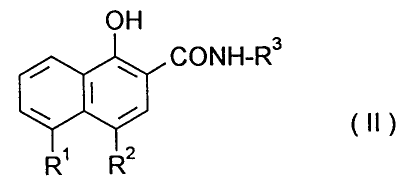
- C-1:: R¹, R² = H; R³ = 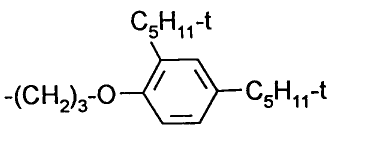
- C-2:: R¹ = -NHCOOCH₂-CH(CH₃)₂; R² = H; R₃ = -(CH₂)₃-OC₁₂H₂₅
- C-3:: R¹ = H; R² = -OCH₂-CH₂-SO₂CH₃; R³ = -C₁₆H₃₃
- C-4:: R¹ = H; R² = -OCH₂-CONH-(CH₂)₂-OCH₃; R₃ = 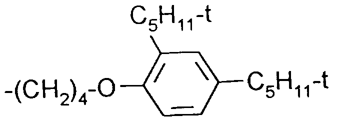
- C-5:: R¹, R² = H; R³ = 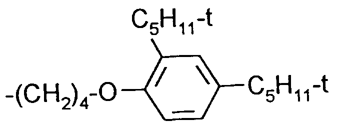
- C-6:: R¹, R² = H; R³ = 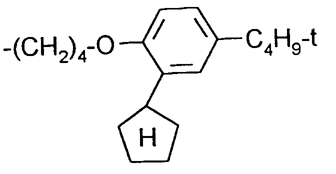
- C-7:: R¹ = H; R² = Cl; R³ = C(C₂H₅)₂-C₂₁H₄₃
- C-8:: R¹ = H; R² = -O-CH₂-CH₂-S-CH(COOH)-C₁₂H₂₅; R³ = Cyclohexyl 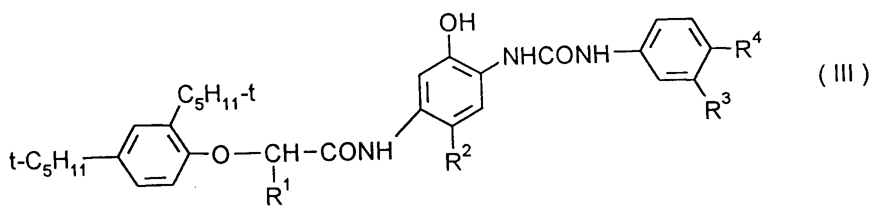
- C-9:: R¹ = -C₄H₉; R² = H; R³ = -CN; R⁴ = Cl
- C-10:: R¹ = -C₄H₉; R² = H; R³ = H; R⁴ = -SO₂-CHF
- C-11:: R¹ = -C₄H₉; R² = 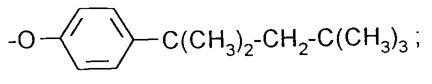 R³ = H; R⁴ = H
- C-12:: R¹ = C₂H₅; R², R³ = H; R⁴ = -SO₂CH₃
- C-13:: R¹ = -C₄H₉; R², R³ = H; R⁴ = -SO₂-C₄H₉
- C-14:: R¹ = -C₄H₉; R² = H; R³ = -CN; R⁴ = -CN
- C-15:: R¹ = -C₄H₉; R², R³ = H; R⁴ = -SO₂-CH₂-CHF₂
- C-16:: R¹ = -C₂H₅; R², R³ = H; R⁴ = -SO₂CH₂-CHF-C₃H₇
- C-17:: R¹ = -C₄H₉; R², R³ = H; R⁴ = F
- C-18:: R¹ = -C₄H₉; R², R³ = H; R⁴ = -SO₂CH₃
- C-19:: R¹ = -C₄H₉; R², R³ = H; R⁴ = -CN 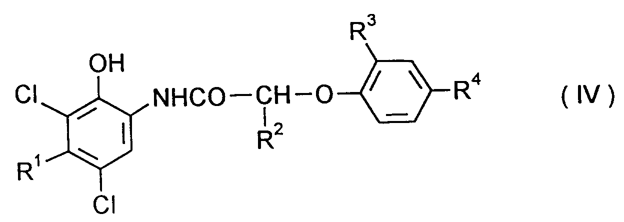
- C-20:: R¹ = -CH₃; R² = -C₂H₅; R³, R⁴ = -C₅H₁₁-t
- C-21:: R¹ = -CH₃; R² = H; R³, R⁴ = -C₅H₁₁-t
- C-22:: R¹, _{,}R² = -C₂H₅; R³, R⁴ = -C₅H₁₁-t
- C-23:: R¹ = -C₂H₅; R² = -C₄H₉; R³, R⁴ = -C₅H₁₁-t
- C-24:: R¹ = -C₂H₅; R² = -C₄H₉; R³, R⁴ = -C₄H₉-t 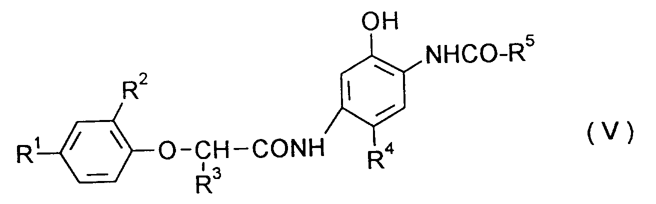
- C-25:: R¹, R² = -C₅H₁₁-t; R³ = -C₄H₉; R⁴ = H; R⁵ = -C₃H₇
- C-26:: R¹ = -NHSO₂-C₄H₉; R² = H; R³ = -C₁₂H₂₅; R⁴ = Cl; R⁵ = Phenyl
- C-27:: R¹, R² = -C₅H₁₁-t; R³ = -C₃H₇-i; R⁴ = Cl; R⁵ = Pentafluorphenyl
- C-28:: R¹ = -C₅H₁₁-t; R² = Cl; R³ = -C₆H₁₃; R⁴ = Cl; R⁵ = -2-Chlorphenyl

In bevorzugten Ausführungsformen der Erfindung werden für die Erzeugung des blaugrünen Teilfarbenbildes phenolische Cyankuppler verwendet, die in 2-Stellung einen ballastierten Acylaminorest und in 5-Stellung eine Ethylgruppe tragen, z.B. Kuppler der Formel IV, worin R¹ Ethyl und R², R³ und R⁴ Alkyl bedeuten.

Farbkuppler zur Erzeugung des purpurnen Teilfarbenbildes sind in der Regel Kuppler vom Typ des 5-Pyrazolons, des Indazolons oder der Pyrazolocole; geeignete Beispiele hierfür sind 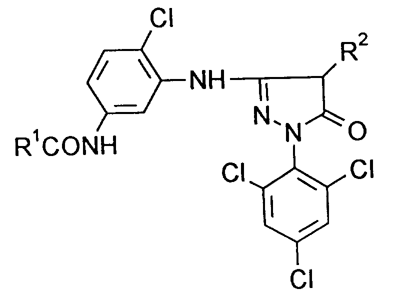
M-1: R¹ = 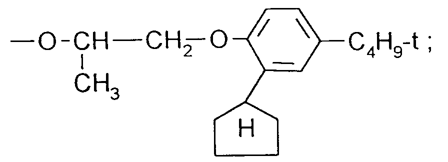 R² = H
M-2: R¹ = 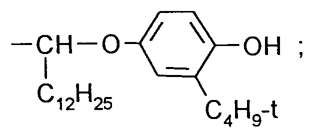 R² = H
M-3: R¹ = -C₁₃H₂₇; R² = H
M-4: R¹ = -CO₁₆H₃₃; R² = H
M-5: R¹ = -C₁₃H₂₇; R² = 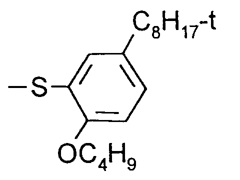
M-6: R¹ = 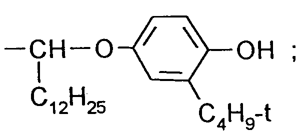 R² = 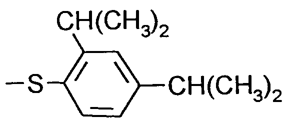
M-7: R¹ = -C₉H₁₉; R² = 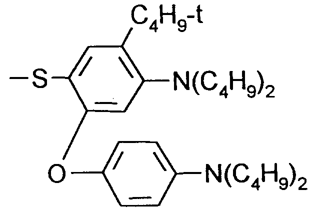
M-8: R¹ = 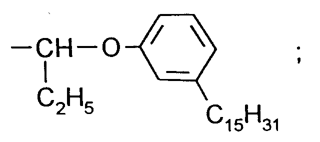 R² = 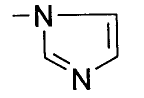
M-9: 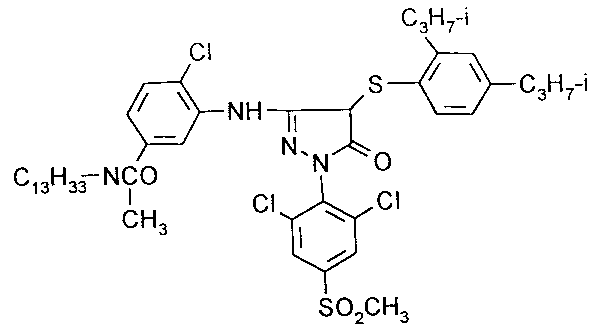
M-10: 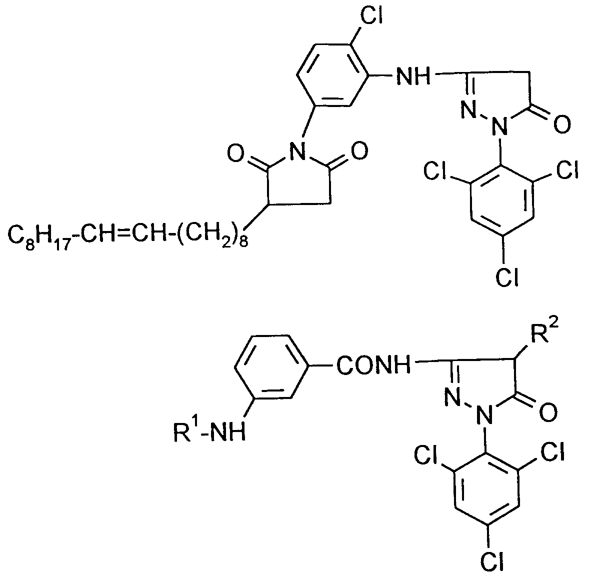
M-11: R¹ = 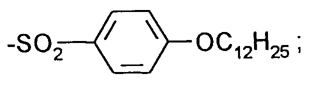 R² = H
M-12: R¹ = 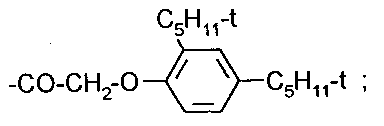 R² = H
M-13: R¹ = 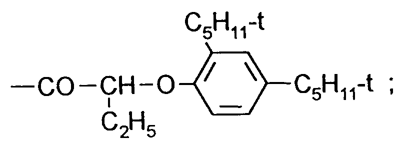 R² = H
M-14: R¹ = 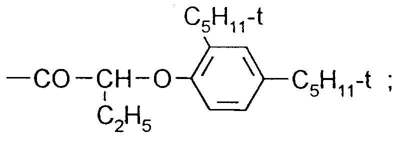 R² = 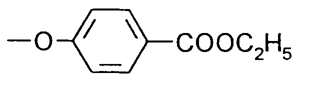
M-15: 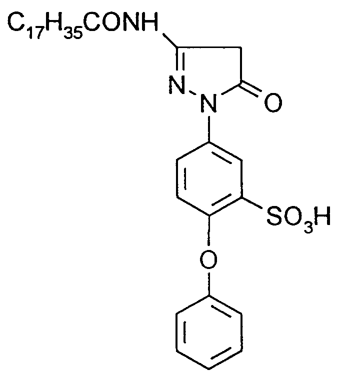
M-16 : 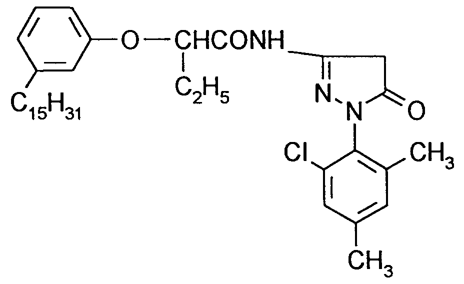
M-17: 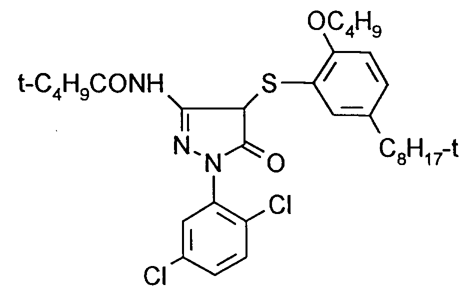 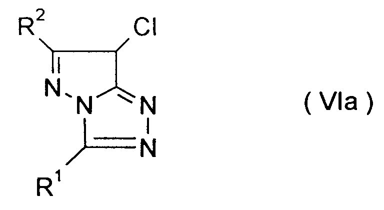
M-18: R¹ = 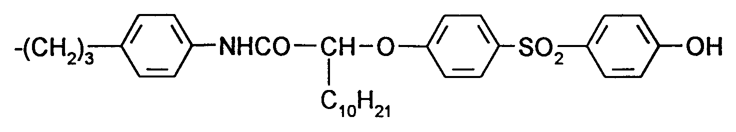 R² = -CH₃
M-19: 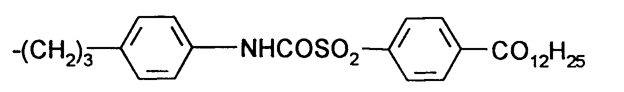 R² = -CH₃
M-20: R¹ = 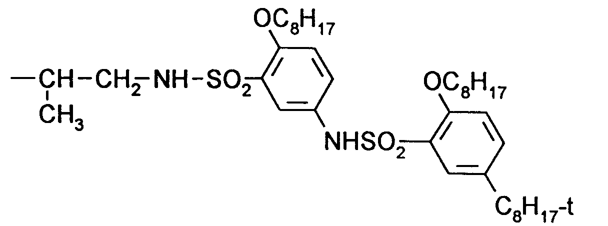 R² = -C₄H₉-t
M-21: R¹ = 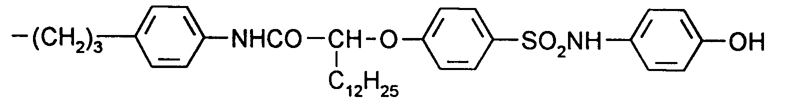 R² = -CH₃
M-22: R¹ = 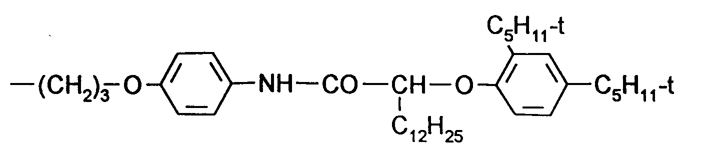 R² = -CH₃
M-23: R¹ = 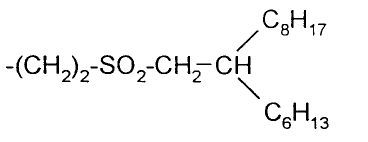 R² = -CH₃
M-24: R¹ = 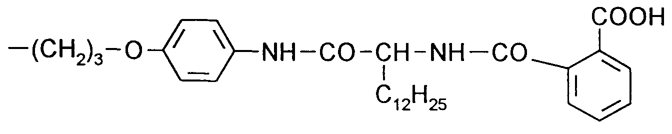 R² = -CH₃ 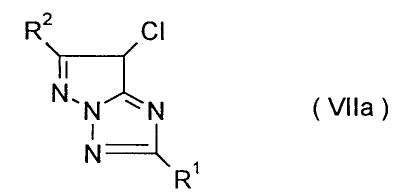
M-25: R¹ = 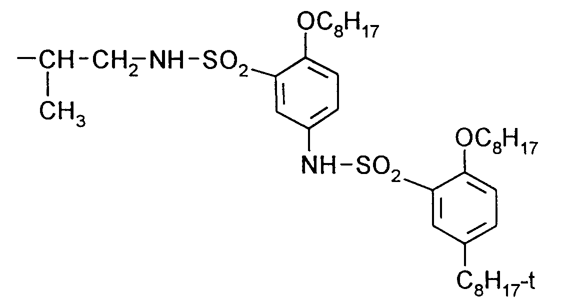 R² = -CH₃
M-26: R¹ = 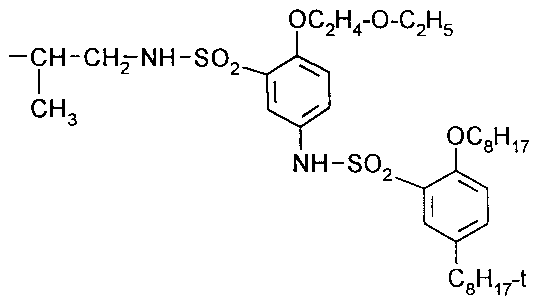 R² = -CH₃
M-27: R¹ = 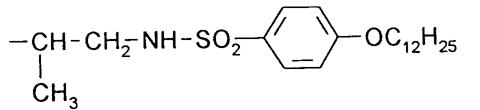 R² = -C₃H₇-i
M-28: R¹ = 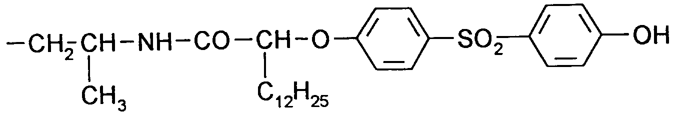 R² = -CH₃
M-29: R¹ = -C₃H₇-i R² = 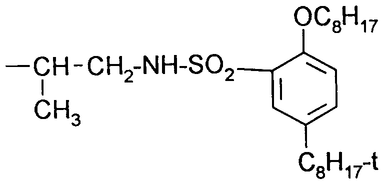
M-30:

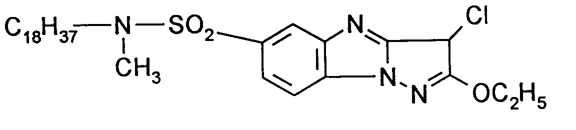

Pyrazoloazolkuppler der allgemeinen Formel VI und VII 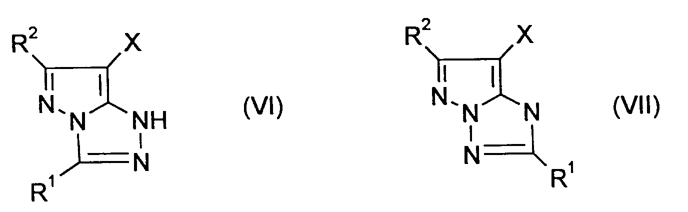
sind beispielsweise in US-A-3 725 067 und US-A-4 540 654 beschrieben. In den Formeln VI und VII bedeuten:
- X: H oder eine unter den Bedingungen der Farbentwicklung freisetzbare Gruppe;
- R¹,R²: H, Alkyl, Aralkyl, Aryl, Alkoxy, Aroxy, Alkylthio, Arylthio, Amino, Anilino, Acylamino, Cyano, Alkoxycarbonyl, Carbamoyl, Sulfamoyl, wobei diese Reste weiter substituiert sein können.

In besonders bevorzugten Ausfürhungsformen der Erfindung werden für die Erzeugung des purpurnen Teilfarbenbildes Pyrazoloazolkuppler einer der Formeln VI und VII verwendet, in denen R² Tertiäralkyl bedeutet.

Bei den Farbkupplern kann es sich um 4-Äquivalentkuppler, aber auch um 2-Äquivalentkuppler handeln. Letztere leiten sich von den 4-Äquivalentkupplern dadurch ab, daß sie in der Kupplungsstelle einen Substituenten enthalten, der bei der Kupplung abgespalten wird. Zu den 2-Äquivalentkupplern sind solche zu rechnen, die farblos sind, als auch solche, die eine intensive Eigenfarbe aufweisen, die bei der Farbkupplung verschwindet bzw. durch die Farbe des erzeugten Bildfarbstoffes ersetzt wird (Maskenkuppler), und die Weißkuppler, die bei Reaktion mit Farbentwickleroxidationsprodukten im wesentlichen farblose Produkte ergeben. Zu den 2-Äquivalentkupplern sind ferner solche Kuppler zu rechnen, die in der Kupplungsstelle einen abspaltbaren Rest enthalten, der bei Reaktion mit Farbentwickleroxidationsprodukten in Freiheit gesetzt wird und dabei entweder direkt oder nachdem aus dem primär abgespaltenen Rest eine oder mehrere weitere Gruppen abgespalten worden sind (z.B. DE-A-27 03 145, DE-A-28 55 697, DE-A-31 05 026, DE-A-33 19 428), eine bestimmte erwünschte fotografische Wirksamkeit entfaltet, z.B. als Entwicklungsinhibitor oder -accelerator. Beispiele für solche 2-Äquivalentkuppler sind die bekannten DIR-Kuppler wie auch DAR-bzw. FAR-Kuppler.

DIR-Kuppler, die Entwicklungsinhibitoren vom Azoltyp, z.B. Triazole und Benzotriazole freisetzen, sind in DE-A-24 14 006, 26 10 546, 26 59 417, 27 54 281,28 42 063, 36 26 219, 36 30 564, 36 36 824, 36 44 416 beschrieben. Weitere Vorteile für die Farbwiedergabe, d.h. Farbtrennung und Farbreinheit, und für die Detailwiedergabe, d.h. Schärfe und Körnigkeit, sind mit solchen DIR-Kupplern zu erzielen, die z.B. den Entwicklungsinhibitor nicht unmittelbar als Folge der Kupplung mit einem oxidierten Farbentwickler abspalten, sondern erst nach einer weiteren Folgereaktion, die beispielsweise mit einer Zeitsteuergruppe erreicht wird. Beispiele dafür sind in DE-A-28 55 697, 32 99 671, 38 18 231, 35 18 797, in EP-A-0 157 146 und 0 204 175, in US-A-4 146 396 und 4 438 393 sowie in GB-A-2 072 363 beschrieben.

Zur Steigerung der Empfindlichkeit, des Kontrastes und der maimalen Dichte können vor allem DAR- bzw. FAR-Kuppler eingesetzt werden, die einen Entwicklungsbeschleuniger oder ein Schleiermittel abspalten. Verbindungen dieser Art sind beispielsweise in DE-A-25 34 466, 32 09 110, 33 33 355, 34 10 616, 34 29 545, 34 41 823, in EP-A-0 089 834, 0 110 511, 0 118 087, 0 147 765 und in US-A-4 618 572 und 4 656 123 beschrieben.

Als Beispiel für den Einsatz von BAR-Kuppler (Bleach Accelerator Releasing Coupler) wird auf EP-A-193 389 verwiesen.

Es kann vorteilhaft sein, die Wirkung einer aus einem Kuppler abgespaltenen fotografisch wirksamen Gruppe dadurch zu modifizieren, daß eine intermolekulare Reaktion dieser Gruppe nach ihrer Freisetzung mit einer anderen Gruppe gemäß. DE-A-35 06 805 eintritt.

Der abspaltbare Rest kann auch ein Ballastrest sein, so daß bei der Reaktion mit Farbentwickleroxidationsprodukten Kupplungsprodukte erhalten werden, die diffusionsfähig sind oder zumindest eine schwache bzw. eingeschränkte Beweglichkeit aufweisen (US-A-4 420 556).

Das Material kann weiterhin von Kupplern verschiedene Verbindungen enthalten, die beispielsweise einen Entwicklungsinhibitor, einen Entwicklungsbeschleuniger, einen Bleichbeschleuniger, einen Entwickler, ein Silberhalogenidlösungsmittel, ein Schleiermittel oder ein Antischleiermittel in Freiheit setzen können, beispielsweise sogenannte DIR-Hydrochinone und andere Verbindungen, wie sie beispielsweise in US-A-4 636 546, 4 345 024, 4 684 604 und in DE-A-31 45 640, 25 15 213, 24 47 079 und in EP-A-198 438 beschrieben sind. Diese Verbindungen erfüllen die gleiche Funktion wie die DIR-, DAR- oder FAR-Kuppler, außer daß sie keine Kupplungsprodukte bilden.

Hochmolekulare Farbkuppler sind beispielsweise in DE-C-1 297 417, DE-A-24 07 569, DE-A-31 48 125, DE-A-32 17 200, DE-A-33 20 079, DE-A-33 24 932, DE-A-33 31 743, DE-A-33 40 376, EP-A-27 284, US-A-4 080 211 beschrieben. Die hochmolekularen Farbkuppler werden in der Regel durch Polymerisation von ethylenisch ungesättigten monomeren Farbkupplern hergestellt. Sie können aber auch durch Polyaddition oder Polykondensation erhalten werden.

Die Einarbeitung der Kuppler oder anderer Verbindungen in Silberhalogenidemulsionsschichten kann in der Weise erfolgen, daß zunächst von der betreffenden Verbindung eine Lösung, eine Dispersion oder eine Emulsion hergestellt und dann der Gießlösung für die betreffende Schicht zugefügt wird. Die Auswahl des geeigneten Lösungs- oder Dispersionsmittels hängt von der jeweiligen Löslichkeit der Verbindung ab.

Methoden zum Einbringen von in Wasser im wesentlichen unlöslichen Verbindungen durch Mahlverfahren sind beispielsweise in DE-A-26 09 741 und DE-A-26 09 742 beschrieben.

Hydrophobe Verbindungen können auch unter Verwendung von hochsiedenden Lösungsmitteln, sogenannten Ölbildnern, in die Gießlösung eingebracht werden. Entsprechende Methoden sind beispielsweise in US-A-2 322 027, US-A-2 801 170, US-A-2 801 171 und EP-A-O 043 037 beschrieben. Geeignete Ölbildner für die erfindungsgemäßen Magentakuppler sind beispielsweise in DE-A-39 18 547 beschrieben.

Anstelle der hochsiedenden Lösungsmittel können Oligomere oder Polymere, sogenannte polymere Ölbildner Verwendung finden.

Die Verbindungen können auch in Form beladener Latices in die Gießlösung eingebracht werden. Verwiesen wird beispielsweise auf DE-A-25 41 230, DE-A-25 41 274, DE-A-28 35 856, EP-A-O 014 921, EP-A-0 069 671, EP-A-O 130 115, US-A-4 291 113.

Die diffusionsfeste Einlagerung anionischer wasserlöslicher Verbindungen (z.B. von Farbstoffen) kann auch mit Hilfe von kationischen Polymeren, sogenannten Beizenpolymeren erfolgen.

Geeignete Ölbildner sind z.B. Phthalsäurealkylester, Phosphonsäureester, Phosphorsäureester, Citronensäureester, Benzoesäureester, Amide, Fettsäureester, Trimesinsäureester, Alkohole, Phenole, Anilinderivate und Kohlenwasserstoffe.

Beispiele für geeignete Ölbildner sind Dibutylphthalat, Dicyclohexylphthalat, Di-2-ethylhexylphthalat, Decylphthalat, Triphenylphosphat, Tricresylphosphat, 2-Ethylhexyldiphenylphosphat, Tricyclohexylphosphat, Tri-2-ethylhexylphosphat, Tridecylphosphat, Tributoxyethylphosphat, Trichlorpropylphosphat, Di-2-ethylhexylphenylphosphat, 2-Ethylhexylbenzoat, Dodecylbenzoat, 2-Ethylhexyl-p-hydroxybenzoat, Diethyldodecanamid, N-Tetradecylpyrrolidon, Isostearylalkohol, 2,4-Di-t-amylphenol, Dioctylacelat, Glycerintributyrat, Isostearyllactat, Trioctylcitrat, N,N-Dibutyl-2-butoxy-5-t-octylanilin, Paraffin, Dodecylbenzol und Diisopropylnaphthalin.

Jede der unterschiedlich sensibilisierten, lichtempfindlichen Schichten kann aus einer einzigen Schicht bestehen oder auch zwei oder mehr Silberhalogenidemulsionsteilschichten umfassen (DE-C-1 121 470). Dabei sind rotempfindliche Silberhalogenidemulsionsschichten dem Schichtträger häufig näher angeordnet als grünempfindliche Silberhalogenidemulsionsschichten und diese wiederum näher als blauempfindliche, wobei sich im allgemeinen zwischen grünempfindlichen Schichten und blauempfindlichen Schichten eine nicht lichtempfindliche gelbe Filterschicht befindet.

Bei geeignet geringer Eigenempfindlichkeit der grün-bzw. rotempfindlichen Schichten kann man unter Verzicht auf die Gelbfilterschicht andere Schichtanordnungen wählen. Erfindungsgemäß bevorzugt ist ein Aufzeichnungsmaterial, das auf einem lichtreflektirenden Träger als unterste lichtempfindliche Schicht eine blauempfindliche Schicht mit einen Gelbkuppler, darüber eine grünempfindliche Schicht mit der erfindungsgemäßen Kombination aus einen Pyrazoloazolkuppler und einem Lichtstabilisator, und als oberste lichtempfindliche Schicht eine rotempfindliche Schicht mit einem phenolischen Cyankuppler enthält.

Die in der Regel zwischen Schichten unterschiedlicher Spektralempfindlichkeit angeordneten nicht lichtempfindlichen Zwischenschichten können Mittel enthalten, die eine unerwünschte Diffusion von Entwickleroxidationsprodukten aus einer lichtempfindlichen in eine andere lichtempfindliche Schicht mit unterschiedlicher spektraler Sensibilisierung verhindern.

Geeignete Mittel, die auch Scavenger oder EOP-Fänger genannt werden, werden in Research Disclosure 17 643 (Dez. 1978), Kapitel VII, 17 842 (Feb. 1979) und 18 716 (Nov. 1979), Seite 650 sowie in EP-A-0 069 070, 0 098 072, 0 124 877, 0 125 522 beschrieben.

Beispiele für besonders geeignete Verbindungen sind: 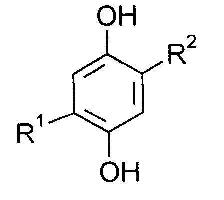
- R¹, R² =: -C₈H₁₇-
-C₁₂H₂₅-a
-C₆H₁₃-t 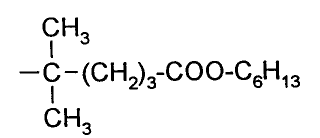 -C₈H₁₇-s
-C₁₅H₃₁

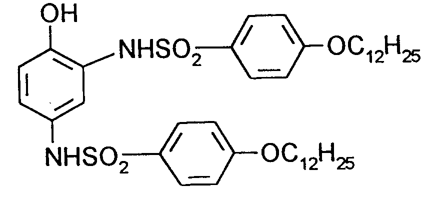

Liegen mehrere Teilschichten gleicher spektraler Sensibilisierung vor, so können sich diese hinsichtlich ihrer Zusammensetzung, insbesondere was Art und Menge der Silberhalogenidkörnchen betrifft, unterscheiden. Im allgemeinen wird die Teilschicht mit höherer Empfindlichkeit von Träger entfernter angeordnet sein als die Teilschicht mit geringerer Empfindlichkeit. Teilschichten gleicher spektraler Sensibilisierung können zueinander benachbart oder durch andere Schichten, z.B. durch Schichten anderer spektraler Sensibilisierung getrennt sein. So können z.B. alle hochempfindlichen und alle niedrigempfindlichen Schichten jeweils zu einem Schichtpaket zusammengefaßt sein (DE-A-19 58 709, DE-A-25 30 645, DE-A-26 22 922).

Das fotografische Material kann weiterhin UV-Licht absorbierende Verbindungen, Weißtöner, Abstandshalter, Filterfarbstoffe, Formalinfänger, Lichtschutzmittel, Antioxidantien, D_{Min}-Farbstoffe, Zusätze zur Verbesserung der Farbstoff-, Kuppler- und Weißenstabilisierung sowie zur Verringerung des Farbschleiers, Weichmacher (Latices), Biocide und anderes enthalten.

UV-Licht absorbierende Verbindungen sollen einerseits die Bildfarbstoffe vor dem Ausbleichen durch UV-reiches Tageslicht schützen und andererseits als Filterfarbstoffe das UV-Licht im Tageslicht bei der Belichtung absorbieren und so die Farbwiedergabe eines Films verbessern. Üblicherweise werden für die beiden Aufgaben Verbindungen unterschiedlicher Struktur eingesetzt. Beispiele sind arylsubstituierte Benzotriazolverbindungen (US-A-3 533 794), 4-Thiazolidonverbindungen (US-A-3 314 794 und 3 352 681), Benzophenonverbindungen (JP-A-2784/71), Zimtsäureesterverbindungen (US-A-3 705 805 und 3 707 375), Butadienverbindungen (US-A-4 045 229) oder Benzoxazolverbindungen (US-A-3 700 455).

Es können auch ultraviolettabsorbierende Kuppler (wie Blaugrünkuppler des α-Naphtholtyps) und ultraviolettabsorbierende Polymere verwendet werden. Diese Ultraviolettabsorbentien können durch Beizen in einer speziellen Schicht fixiert sein.

Für sichtbares Licht geeignete Filterfarbstoffe umfassen Oxonolfarbstoffe, Hemioxonolfarbstoffe, Styrylfarbstoffe, Merocyaninfarbstoffe, Cyaninfarbstoffe und Azofarbstoffe. Von diesen Farbstoffen werden Oxonolfarbstoffe, Hemioxonolfarbstoffe und Merocyaninfarbstoffe besonders vorteilhaft verwendet.

Geeignete Weißtöner sind z.B. in Research Disclosure 17 643 (Dez. 1978), Kapitel V, in US-A-2 632 701, 3 269 840 und in GB-A-852 075 und 1 319 763 beschrieben.

Bestimmte Bindemittelschichten, insbesondere die vom Träger am weitesten entfernte Schicht, aber auch gelegentlich Zwischenschichten, insbesondere, wenn sie während der Herstellung die vom Träger am weitesten entfernte Schicht darstellen, können fotografisch inerte Teilchen anorganischer oder organischer Natur enthalten, z.B. als Mattierungsmittel oder als Abstandshalter (DE-A-33 31 542, DE-A-34 24 893, Research Disclosure 17 643, (Dez. 1978), Kapitel XVI).

Der mittlere Teilchendurchmesser der Abstandshalter liegt insbesondere im Bereich von 0,2 bis 10 m. Die Abstandshalter sind wasserunlöslich und können alkaliunlöslich oder alkalilöslich sein, wobei die alkalilöslichen im allgemeinen im alkalischen Entwicklungsbad aus dem fotografischen Material entfernt werden. Beispiele für geeignete Polymere sind Polymethylmethacrylat, Copolymere aus Acrylsäure und Methylmethacrylat sowie Hydroxypropylmethylcellulosehexahydrophthalat.

Zusätze zur Verbesserung der Farbstoff-, Kuppler- und Weißenstabilität sowie zur Verringerung des Farbschleiers (Research Disclosure 17 643 (Dez. 1978), Kapitel VII) können den folgenden chemischen Stoffklassen angehören: Hydrochinone, 6-Hydroxychromane, 5-Hydroxycumarane, Spirochromane, Spiroindane, p-Alkoxyphenole, sterische gehinderte Phenole, Gallussäurederivate, Methylendioxybenzole, Aminophenole, sterisch gehinderte Amine, Derivate mit veresterten oder verätherten phenolischen Hydroxylgruppen, Metallkomplexe.

Verbindungen, die sowohl eine sterisch gehinderte Amin-Partialstruktur als auch eine sterisch gehinderte Phenol-Partialstruktur in einem Molekül aufweisen (US-A-4 268 593), sind besonders wirksam zur Verhinderung der Beeinträchtigung von gelben Farbbildern als Folge der Entwicklung von Wärme, Feuchtigkeit und Licht. Um die Beeinträchtigung von purpurroten Farbbildern, insbesondere ihre Beeinträchtigung als Folge der Einwirkung von Licht, zu verhindern, sind beispielsweise Spiroindane (JP-A-159 644/81) und Chromane (JP-A-89 835/80) besonders wirksam.

Beispiele besonders geeigneter Verbindungen sind: 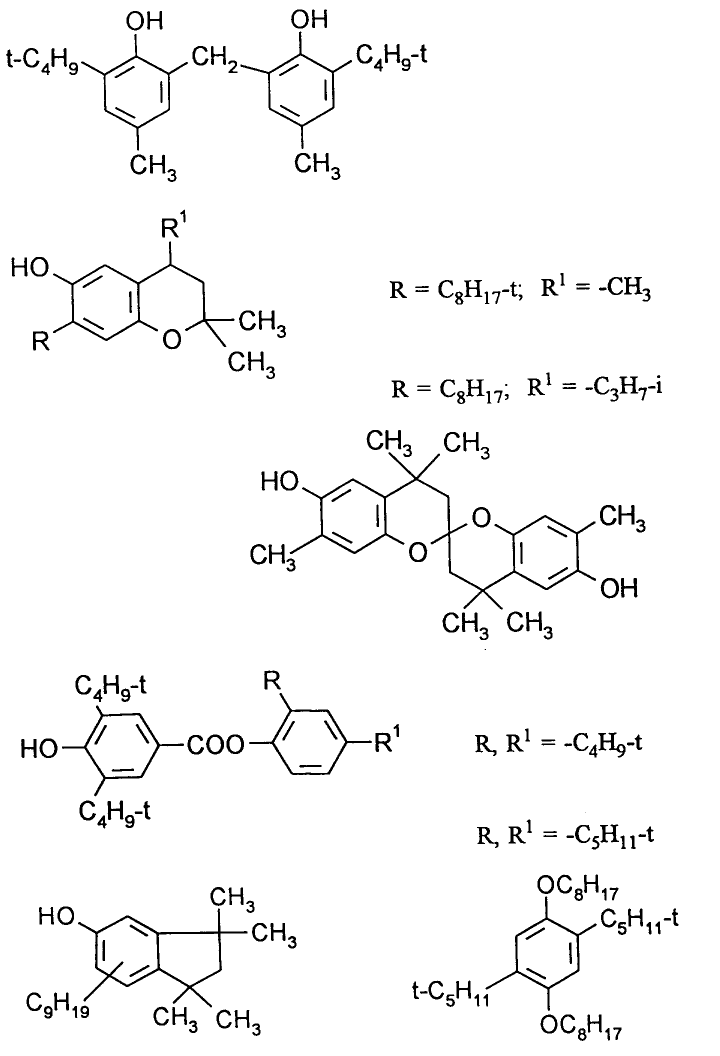 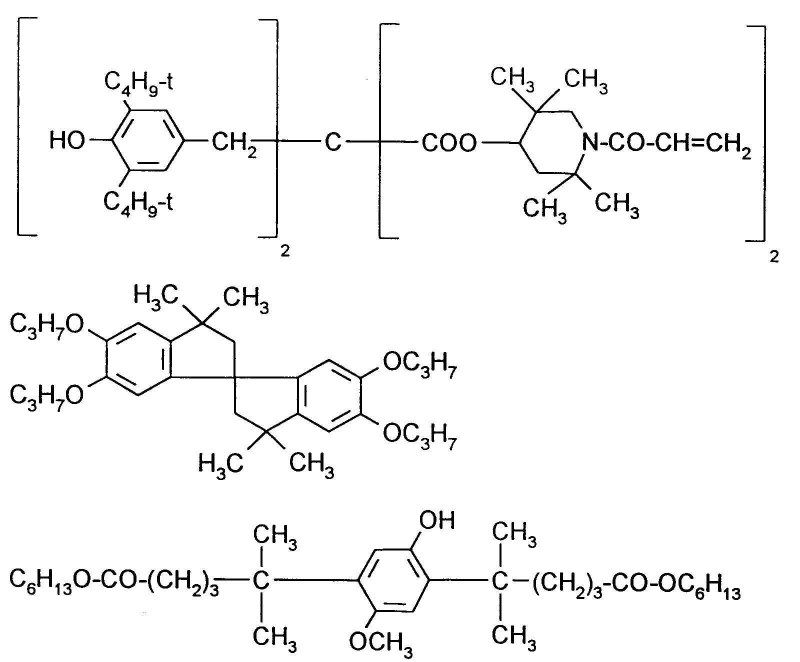
sowie die als EOP-Fänger aufgeführten Verbindungen.

Farbfotografische Aufzeichnungsmaterialien werden üblicherweise durch Entwickeln, Bleichen, Fixieren und Wässern oder durch Entwickeln, Bleichen, Fixieren und Stabilisieren ohne nachfolgende Wässerung verarbeitet, wobei Bleichen und Fixieren zu einem Verarbeitungsschritt zusammengefaßt sein können. Als Farbentwicklerverbindung lassen sich sämtliche Entwicklerverbindungen verwenden, die die Fähigkeit besitzen, in Form ihres Oxidationsproduktes mit Farbkupplern zu Azomethin-bzw. Indophenolfarbstoffen zu reagieren. Geeignete Farbentwicklerverbindungen sind aromatische, mindestens eine primäre Aminogruppe enthaltende Verbindungen vom p-Phenylendiamintyp, beispielsweise N,N-Dialkyl-p-phenylendiamine wie N,N-Diethyl-p-phenylendiamin, 1-(N-Ethyl-N-methansulfonamidoethyl)-3-methyl-p-phenylendiamin, 1-(N-Ethyl-N-hydroxyethyl)-3-methyl-p-phenylendiamin und 1-(N-Ethyl-N-methoxyethyl)-3-methyl-p-phenylendiamin. Weitere brauchbare Farbenwickler sind beispielsweise in J. Amer. Chem. Soc. 73, 3106 (1951) und G. Haist, Modern Photographic Processing, 1979, John Wiley and Sons, New York, Seite 545 ff. beschrieben.

Nach der Farbentwicklung kann ein saures Stoppbad oder eine Wässerung folgen.

Üblicherweise wird das Material unmittelbar nach der Farbentwicklung gebleicht und fixiert. Als Bleichmittel können z.B. Fe(III)-Salze und Fe(III)-Komplexsalze wie Ferricyanide, Dichromate, wasserlösliche Kobaltkomplexe verwendet werden. Besonders bevorzugt sind Eisen-(III)-Komplexe von Aminopolycarbonsäuren, insbesondere z.B. von Ethylendiamintetraessigsäure, Propylendiamintetraessigsäure, Diethylentriaminpentaessigsäure, Nitrilotriessigsäure, Iminodiessigsäure, N-Hydroxyethyl-ethylendiaminthessigsaure, Alkyliminodicarbonsäuren und von entsprechenden Phosphonsäuren. Geeignete als Bleichmittel sind weiterhin Persulfate und Peroxide, z.B. Wasserstoffperoxid.

Auf das Bleichfixierbad oder Fixierbad folgt meist eine Wässerung, die als Gegenstromwässerung ausgeführt ist oder aus mehreren Tanks mit eigener Wasserzufuhr besteht.

Günstige Ergebnisse können bei Verwendung eines darauf folgenden Schlußbades, das keinen oder nur wenig Formaldehyd enthält, erhalten werden.

Die Wässerung kann aber durch ein Stabilisierbad vollständig ersetzt werden, das üblicherweise im Gegenstrom geführt wird. Dieses Stabilisierbad übernimmt bei Formaldehydzusatz auch die Funktion eines Schlußbades.

Bei Farbumkehrmaterialien erfolgt zunächst eine Entwicklung mit einem SchwarzWeiß-Entwickler, dessen Oxidationsprodukt nicht zur Reaktion mit den Farbkupplern befähigt ist. Es schließt sich eine diffuse Zweitbelichtung und dann Entwicklung mit einem Farbenwickler, Bleichen und Fixieren an.

### Beispiel

Gelb-Einzelgüsse für ein zur Schnellverarbeitung geeignetes farbfotografisches Aufzeichnungsmaterial werden hergestellt, indem auf eine Seite eines Schichtträgers aus beiderseits mit Polyethylen kaschiertem Papier jeweils 4 Schichten in der angegebenen Reihenfolge aufgetragen werden.

Die Gewichtsangaben beziehen sich auf 1 m², für den Silberhalogenidauftrag wird die entsprechende Menge AgNO₃ angegeben.

### Prüfmaterial 1: Vergleich

- Schicht 1:: Substrierschicht mit 0,2 g Gelatine
- Schicht 2:: blauempfindliche Silberhalogenidemulsion aus 99,5 mol-% AgCl und 0,5 mol-% AgBr, mittlerer Korndurchmesser 0,8 µm, entsprechend 0,46 g AgNO₃ 0,5 g Gelbkuppler XY-1 0,2 g Weißkuppler XW-1 0,5 g Polyester aus Adipinsäure, Butandiol-1,3-diol und Hexan-1,6-diol
- Schicht 3:: 1,1 g Gelatine 0,06 g Dioctylhydrochinon 0,06 g Di-n-butylphthalat
- Schicht 4:: 0,9 g Gelatine 0,3 g Soforthärtungsmittel CAS Reg. No. 65411-60-1

In Schicht 2 wurden folgende Verbindungen verwendet.

XY-1 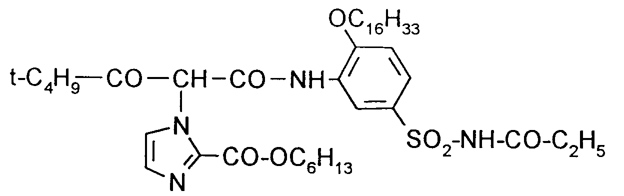
XW-1 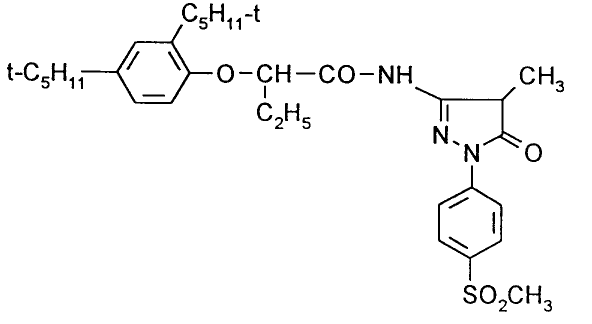
Die Prüfmaterialien 2 bis 5 (erfindungsgemäß) unterscheiden sich von Prüfmaterial 1 dadurch, daß die entsprechende Schicht 2 anstelle des Kupplers XY-1 je 0,5 g eines der erfindungsgemäßen Kuppler 1, 2, 5 und 32 enthalten.

Die erhaltenen Prüfmaterialien wurden hinter einem Graukeil mit blauem Licht und wie nachfolgend angegeben verarbeitet. Anschließend wurde die Stabilität gegenüber Licht und Hitzeeinwirkung bestimmt.
a) Farbentwickler - 45 s - 35°C

| | |
|---|---|
| Triethanolamin | 9,0 g |
| N,N-Diethylhydroxylamin | 6,0 g |
| Diethylenglykol | 0,05 g |
| 3-Methyl-4-amino-N-ethyl-N-methansulfonamidoethyl-anilid-sulfat | 6,0 g |
| Kaliumsulfat | 0,2 g |
| Triethylenglykol | 0,05 g |
| Kaliumcarbonat | 22,0 g |
| Kaliumhydroxid | 0,4 g |
| Ethylendiamintetraessigsäure-di-Na-Salz | 2,2 g |
| auffüllen mit Wasser auf 1.000 ml; pH 9,2. | |

b) Bleichfixierbar - 45 s - 35°C

| | |
|---|---|
| Ammoniumthiosulfat | 75 g |
| Natriumhydrogensulfit | 13,5 g |
| Ammoniumacetat | 2,0 g |
| Ethylendiamintetraessigsäure (Eisen-Ammonium-Salz) | 57,0 g |
| Ammoniak 25 %ig | 9,5 g |
| Essigsäure | 9,0 g |
| auffüllen mit Wasser auf 1.000 ml; pH 5,5. | |

c) Wässern - 2 min - 33°C Man erhält folgendes Ergebnis:

| Prüfmaterial | Dₘₐₓ | Lightfading | Darkfading |
|---|---|---|---|
| 1 | 2,09 | -45 % | -19 % |
| 2 | 1,67 | -22 % | ± 0 % |
| 3 | 1,29 | -33 % | ± 0 % |
| 4 | 1,59 | -25 % | ± 0 % |
| 5 | 1,36 | -40 % | - 5 % |

Als Lightfading wird der relative Dichterückgang bei Maximaldichte Dₘₐₓ nach Bestrahlung im Xenotestgerät (9,6 x 10⁶ Luxh) bezeichnet.

Als Darkfading wird der relative Dichterückgang unter Hitzeeinwirkung ohne Bestrahlung bei 90°C über 8 Tage bezeichnet.

Das Ergebnis zeigt, daß die aus den erfindungsgemäßen Gelbkupplern erhaltenen Gelbfarbstoffe überlegene Darkfadingstabilität bei meist ebenfalls günstigerer Lichtstabilität aufweisen.

Prüfmaterial 2 zeigt außerdem gegenüber Prüfmaterial 1 eine verbesserte Absorption, wie anhand der Werte für λₘₐₓ und die Halbbandbreite zu erkennen ist: Das Absorptionsmaximum liegt zwar um 8 nm langwellig verschoben, doch liegt die Halbbandbreite durch einen steileren Abfall der langwelligen Flanke bei einem um 7 nm kleineren λ-Wert.

| Prüfmaterial | λₘₐₓ | λ_{HBB} |
|---|---|---|
| 1 | 444 nm | 515 nm |
| 2 | 452 nm | 508 nm |

## Patentansprüche

1. Gelbkuppler der Formel I 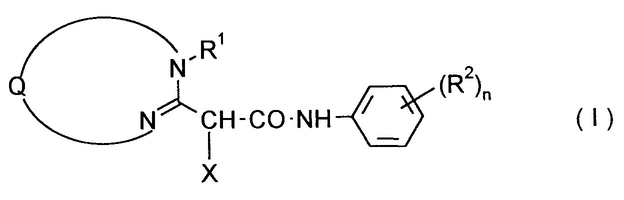 worin bedeuten
Q einen Rest zur Vervollständigung eines 4-Pyrimidonringes;
X einen bei der chromogenen Entwicklung abspaltbaren Rest;
R¹ Alkyl mit 1 bis 6 C-Atomen;
R² Halogen, -CF₃, Alkyl, Aryl, Alkoxy, Aryloxy, Alkylthio, Arylthio, Acylamino, Sulfonamido, Sulfuramido, Carbamoyl, Alkoxycarbonyl, Sulfonyl, Sulfamoyl oder einen heterocyclischen Rest, wobei zwei benachbarte Reste R² einen Ring vervollständigen können;
n eine ganze Zahl von 1 bis 4.

2. Gelbkuppler nach Anspruch 1, dadurch gekennzeichnet, daß in Formel I der durch Q dargestellte Rest zur Vervollständigung eines 4-Pyrimidonringes eine Gruppe der Formel 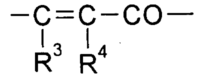
ist, worin
R³ und R⁴ einen Rest zur Vervollständigung eines 5- bis 7-gliedrigen Ringes bedeuten, oder worin einer der Reste R³ und R⁴ für Wasserstoff Alkyl, Aryl oder Alkoxy, der andere für -CN, Acylamino, Carbamoyl, Alkoxycarbonyl, Sulfonyl oder eine Phosphorestergruppe steht.

3. Gelbkuppler nach Anspruch 2, dadurch gekennzeichnet, daß in Formel I der durch X dargestellte abspaltbare Rest ein 1,2,3- oder 1,2,4-Triazolrest ist.

4. Farbfotografisches Aufzeichnungsmaterial mit mindestens einer lichtempfindlichen Silberhalogenidemulsionsschicht, der ein Gelbkuppler zugeordnet ist, dadurch gekennzeichnet, daß der Gelbkuppler der Formel I entspricht 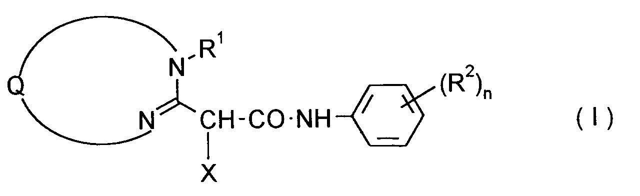 worin bedeuten
Q einen Rest zur Vervollständigung eines 4-Pyrimidonringes;
X einen bei der chromogenen Entwicklung abspaltbaren Rest;
R¹ Alkyl mit 1 bis 6 C-Atomen;
R² Halogen, -CF₃, Alkyl, Aryl, Alkoxy, Aryloxy, Alkylthio, Arylthio, Acylamino, Sulfonamido, Sulfuramido, Carbamoyl, Alkoxycarbonyl, Sulfonyl, Sulfamoyl oder einen heterocyclischen Rest, wobei zwei benachbarte Reste R² einen Ring vervollständigen können;
n eine ganze Zahl von 1 bis 4.

5. Aufzeichnungsmaterial nach Anspruch 4, dadurch gekennzeichnet, daß in Formel I der durch Q dargestellte Rest zur Vervollständigung eines 4-Pyrimidonringes eine Gruppe der Formel 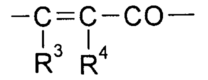 ist, worin
R³ und R⁴ einen Rest zur Vervollständigung eines 5- bis 7-gliedrigen Ringes bedeuten, oder worin einer der Reste R³ und R⁴ für Wasserstoff, Alkyl, Aryl oder Alkoxy, der andere für -CN, Acylamino, Carbamoyl, Alkoxycarbonyl, Sulfonyl oder eine Phosphorestergruppe steht.

6. Aufzeichnungsmaterial nach Anspruch 5, dadurch gekennzeichnet, daß in Formel I der durch X dargestellte abspaltbare Rest ein 1,2,3- oder 1,2,4-Triazolrest ist.

7. Aufzeichnungsmaterial nach einem der Ansprüche 4 bis 6, dadurch gekennzeichnet, daß es mindestens eine blauempfindliche Silberhalogenidemulsionsschicht mit einem Gelbkuppler der Formel I enthält und daß es ferner mindestens eine grünempfindliche Silberhalogenidemulsionsschicht mit einem Magentakuppler einer der Formeln VI und VII und mindestens eine rotempfindliche Silberhalogenidemulsionsschicht mit einem Cyankuppler der Formel IV enthält:
Cyankuppler 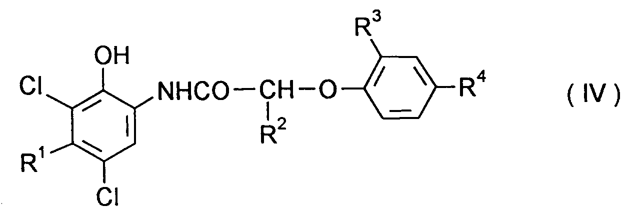 In Formel IV bedeuten
R¹ Alkyl mit mindestens 2 C-Atomen
R², R³, R⁴ Alkyl;
Magentakuppler 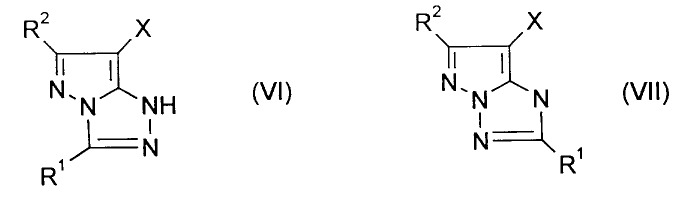 In den Formeln VI und VII bedeuten:
X H oder eine unter den Bedingungen der Farbentwicklung freisetzbare Gruppe;
R¹ H, Alkyl, Aralkyl, Aryl, Alkoxy, Aroxy, Alkylthio, Arylthio, Amino, Anilino, Acylamino, Cyano, Alkoxycarbonyl, Carbamoyl, Sulfamoyl, wobei diese Reste weiter substituiert sein können,
R² Tertiäralkyl.
